(19) Europäisches Patentamt / European Patent Office / Office européen des brevets

(11) **EP 4 557 298 A1**

(12) **EUROPEAN PATENT APPLICATION**

(43) Date of publication:
**21.05.2025 Bulletin 2025/21**

(21) Application number: **23383179.1**

(22) Date of filing: **17.11.2023**

(51) International Patent Classification (IPC):
**G16B 20/10** (2019.01)  **C12Q 1/6886** (2018.01)
**G16B 40/00** (2019.01)

(52) Cooperative Patent Classification (CPC):
**G16B 20/10; C12Q 1/6886; G16B 40/00;**
C12Q 2600/106; C12Q 2600/118; C12Q 2600/156;
G16B 25/10

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Fundación Del Sector Público Estatalcentro Nacional De Investigaciones Oncológicas Carlos III (F.S.P. CNIO)**
**28029 Madrid (ES)**

(72) Inventor: **The designation of the inventor has not yet been filed**

(74) Representative: **Mewburn Ellis LLP**
**Aurora Building**
**Counterslip**
**Bristol BS1 6BX (GB)**

Remarks:
•The application is published incomplete as filed (Rule 68(1) EPC).
•The references to the drawing no. fig. 2 are deemed to be deleted (Rule 56(4)(6) EPC).

(54) **DETECTION OF CHROMOSOMAL INSTABILITY**

(57) Methods of characterising chromosomal instability in a DNA sample obtained from a tumour are described, the methods comprising: obtaining a tumour copy number profile for the sample; quantifying, for each of one or more segments in the copy number profile, a set of copy number features, wherein the set of copy number features consists of copy number features that are associated with respective segments; and determining, for a segment of the one or more segments, a metric indicative of association between one or more signatures of chromosomal instability and the segment using the quantified features for the segment, wherein a signature of chromosomal instability comprises a set of weights for each of a plurality of components each associated with a copy number feature of the set of copy number features, wherein the weights are indicative of the probability that a mutational process associated with the signature generates a segment having characteristics defined by the component.

Fig. 1

## Description

FIELD OF THE DISCLOSURE

The present invention relates to copy number signatures that are associated with different types of chromosomal instability, and in particular to methods of identifying such copy number signatures associated with individual copy number alteration events in a sample.

BACKGROUND

Over 5 million patients are diagnosed each year with extremely deadly cancers. These tumours are characterised by chaotic genomes caused by chromosomal instability (CIN). This chaos fuels cancer development and progression, making these tumours difficult to treat (McGranahan et al. 2021). Accurately detecting CIN is emerging as an important biomarker for prognosis, diagnosis and therapy selection (Macintyre et al. 2018; Drews et al. 2022; Steele et al. 2022). Drews et al. 2022 describes an approach to evaluate the origin and extent of CIN at genome-wide level, based on work in Macintyre et al. 2018. This technology represented a step-change in tackling the deadliest cancer subtypes, which have evaded precision medicine approaches to date.

However, it is still difficult despite this progress to dissect CIN-related cancer evolution and tumour heterogeneity, which is intrinsically linked to tumour progression, and ultimately treatment sensitivity and resistance.

SUMMARY

The present inventors have recognised that current approaches to analysis of patterns of chromosomal instability cannot be directly applied for dissecting CIN from genomically-isolated single copy number events. This limits our ability to dissect genomic patterns caused by historical CIN (sometimes occurring up to 20-years prior to diagnosis) and ongoing CIN (currently operating in a tumour and driving its progression). Mapping each individual copy number event to a specific CIN type is crucial for effectively evaluating CIN-related cancer evolution and tumour heterogeneity, and improving therapeutic targeting. Therefore, the present inventors devised a new approach to characterise copy number instability by associating signatures of chromosomal instability to single events rather than whole copy number profiles. This required designing new copy number features to characterise copy number events that can each be associated with single events, creating a new feature space characterising copy number events.

Thus, according to a first aspect, there is provided a computer-implemented method of characterising chromosomal instability in a DNA sample obtained from a tumour, the method comprising: obtaining a tumour copy number profile for the sample; quantifying, for each of one or more segments in the copy number profile, a set of copy number features, wherein the set of copy number features consists of copy number features that are associated with respective segments; and determining, for a segment of the one or more segments, a metric indicative of association between one or more signatures of chromosomal instability and the segment using the quantified features for the segment, wherein a signature of chromosomal instability comprises a set of weights for each of a plurality of components each associated with a copy number feature of the set of copy number features, wherein the weights are indicative of the probability that a mutational process associated with the signature generates a segment having characteristics defined by the component.

The methods according to the present aspect may have any one or more of the following optional features.

The set of copy number features may comprise or consist of: segment size, copy number change-point, and breakpoint count in one or both flanking regions of a predetermined length. Determining a metric indicative of association between a signature of chromosomal instability and a segment may comprise obtaining a value for each of the plurality of components for the segment. Each component may define characteristics of a segment as a predetermined distribution of values of a copy number feature. Determining a metric indicative of association between a signature of chromosomal instability and a segment may comprise determining the probability of the copy number features of the segment belonging to each of a set of predetermined distribution associated with respective components of the plurality of components.

The set of copy number features for a segment may comprise a summarised value of the breakpoint count in the upstream and downstream flanking regions of a predetermined length around the segment. The summarised value may be the maximum of the breakpoint count in the upstream and downstream flanking regions of a predetermined length around the segment. The predetermined length may be between 2 and 10 Mb, between 3 and 8 Mb, or about 5 Mb.

Each copy number feature may be associated with a plurality of components, and each component may define a characteristic of copy number events using a respective predetermined distribution. The predetermined distribution for a copy number feature that is quasi-continuous (e.g. segment size, copy number changepoint) may be a set of Gaussian distributions. The predetermined distributions for a copy number feature that is a count feature (e.g. breakpoint count in one or both flanking regions of a predetermined length) may be a set of Poisson distributions.

The predetermined distributions associated with the components may be the distributions defined by the parameters in Table 1, or corresponding distributions obtained by fitting mixture models to values for the set of copy number features obtained from copy number profiles from a plurality of tumour samples. The predetermined distributions may comprise between 15 and 25 Gaussian distributions for the segment size, between 5 and 15 Gaussian distributions for the copy number changepoint, and/or between 5 and 15 Poisson distributions for the breakpoint count in one or both flanking regions of a predetermined length.

The set of copy number features may comprise a copy number change-point, wherein the copy number changepoint of a current segment is determined by reference to the upstream segment of the current segment when the copy number changepoint relative to the upstream segment is at or above a predetermined threshold, by reference to the downstream segment of the current segment when the copy number changepoint relative to the upstream segment is below the predetermined threshold and the copy number changepoint relative to the downstream segment is at or above the predetermined threshold, and by reference to a reference copy number value when the copy number changepoints relative to the upstream and downstream segments are below the predetermined threshold. The reference copy number value may be 2. The predetermined threshold may be -3.

Each of the one or more segments may be a non-diploid segment. Quantifying the set of copy number features may comprise using unrounded copy number segments. Obtaining a copy number profile may comprise collapsing and merging near diploid segments to a diploid state, wherein near diploid segments are segments that have a copy number within a predetermined distance from 2. The predetermined distance may be 0.1.

Determining a metric indicative of association between a signature of chromosomal instability and a segment using the quantified features for the segment may comprise multiplying probabilities associated with each of the plurality of components for the segment by the corresponding weight in the signature, and summing up the resulting values. The probability associated with a component for the segment may be the probability of a segment with the quantified feature having been drawn from the distribution associated with the component. Determining a metric indicative of association between a signature of chromosomal instability and a segment may comprise, for each of a plurality of signatures, multiplying probabilities associated with each of the plurality of components for the segment by the corresponding weight in the signature, and summing up the resulting values to obtain an exposure for the signature, and normalising the resulting exposures by dividing each exposure by the sum of exposures across the plurality of signatures. Determining a metric indicative of association between a signature of chromosomal instability and a segment using the quantified features for the segment may comprise obtaining probabilities associated with each of the plurality of components for the segment and determining a distance between said probabilities and the weights of the signature. The distance may be a cosine distance or a Euclidian distance.

The method may comprise determining the exposure of the sample to the signature by: obtaining, for each component, the sum of probabilities associated with the respective component for each of a plurality of segments in the copy number profile; and

determining a linear combination of one or more signatures comprising the signature that satisfies the equation

$$PbC \approx E \times SbC \qquad \text{(Equation 1)}$$

where E is a vector of size n comprising coefficients $E_{1,...,n}$ where $E_i$ is the exposure to signature i; PbC is a vector of size c, each element in the vector representing a sum of probabilities associated with a respective component; and SbC is a matrix of size c by n, each value representing the weight of a component in a signature i.

The method may further comprise assigning a signature of chromosomal instability to a segment of the one or more segments as the signature of the one or more signatures of chromosomal instability with the highest value of the metric for the segment.

The metric for a signature may be a probability that the segment is associated with the signature.

The one or more signatures of chromosomal instability may be selected from those defined in Table 3 or corresponding signatures obtained by quantifying the set of copy number features in a plurality of tumour samples, and identifying one or more signatures likely to result in the copy number profiles of the plurality of tumour samples by nonnegative matrix factorisation.

The one or more signatures may comprise one or more signatures selected from: one or more signatures associated with chromosome missegregation, optionally chromosome missegregation via defective mitosis and/or telomere dysfunction, one or more signatures associated with impaired homologous recombination, one or more signatures associated with tolerance of whole genome duplication, one or more signatures associated with impaired non-homologous end joining, one or more signatures associated with replication stress, and/or one or more signature associated with impaired DNA damage sensing.

The sample may have been obtained from a subject who has bene diagnosed as having cancer. The sequence data may have been obtained using sequencing. The sequencing may be selected from WGS, sWGS, single cell WGS, WES or panel sequencing, or a copy number array.

The method may further comprise determining that the copy number profile comprises a number of non-diploid segments above a predetermined threshold. The copy number profile may comprise at least 15 non-diploid segments.

Obtaining the tumour copy number profile may comprise determining the tumour copy number profile from sequence data. The sequence data may be next-generation sequencing data. Determining the tumour copy number profile may comprise determining a copy number for each of a plurality of genomic bins of a predetermined size. The predetermined size may be at most 100kb, at most 50kb, or about 30kb. The method may comprise obtaining a tumour copy number profile from DNA sequence data comprising a plurality of sequence read, wherein the tumour copy number profile comprises a copy number estimate for each of a plurality of genomic bins comprising a number of mapped reads above a predetermined threshold. The predetermined threshold may be 15 reads.

Also described according to a second aspect is a method of predicting whether a subject with cancer is likely to respond to a therapy, the method comprising: characterising a DNA sample obtained from a tumour of the subject, using the method of any embodiment of the first aspect, as having at least one segment with a metric associated with one or more predetermined signatures of chromosomal instability satisfying a predetermined criterion and/or as having a sample exposure to said signatures satisfying a predetermined criterion, wherein the predetermined signatures comprise a signature associated with response to inhibition of a particular gene, the therapy is a therapy that targets the gene, and when the sample is characterised as having at least one segment with the metric associated with the signature satisfying a predetermined criterion and/or as having a sample exposure to said signature satisfying a predetermined criterion, the subject is likely to respond to the therapy. For example, a signature associated with tolerance to whole genome duplication or PI3K/AKT-mediated tolerance of whole genome duplication (such as e.g. CX4 in Table 3 or a corresponding signature) has been shown to be associated with sensitivity to a therapy that inhibits CCND1. As another example, a signature associated with impaired homologous recombination (such as e.g. CX5 in Table 3 or a corresponding signature) has been shown to be sensitive to a therapy that inhibits PARP1. As another example, a signature associated with replication stress (optionally wherein the signature is further indicative of focal amplifications; such as e.g. CX9 in Table 3 or a corresponding signature) has been shown to be associated with sensitivity to a therapy that inhibits a kinase in a mitogenic pathway (such as EGFR, JAK1, MET, PRKCA, PI3KCA). As another example, a signature associated with replication stress (optionally wherein the signature is further indicative of clustered amplifications, such as e.g. CX13 in Table 3 or a corresponding signature) has been shown to be associated with a therapy that inhibits CDK4.

Also described according to a third aspect is a method of predicting whether a subject with cancer is likely to respond to a therapy, the method comprising: characterising a DNA sample obtained from a tumour of the subject, using the method of any embodiment of the first aspect, as having at least one segment with a metric associated with one or more predetermined signatures of chromosomal instability satisfying a predetermined criterion and/or as having a sample exposure to said signatures satisfying a predetermined criterion, wherein the therapy is a therapy for which resistance to the therapy is associated with amplification of a gene, optionally amplification of an oncogene, when the sample is characterised as having at least one segment with the metric associated with the one or more signatures satisfying a predetermined criterion and/or as having a sample exposure to said signatures satisfying a predetermined criterion, the subject is likely to develop an amplification of the gene and is unlikely to respond to the therapy.

The methods of the second and third aspects may comprise recommending a treatment comprising the therapy to a subject who has been determined as likely to respond to the therapy. The methods of the second and third aspects may comprise recommending a treatment that does not comprise the therapy to a subject who has been determined as unlikely to respond to the therapy.

Methods of the disclosure also encompass treating a subject who has been diagnosed with cancer, the method comprising determining whether the subject is likely to respond to a therapy as described herein and administering the therapy to a subject who has been determined as likely to respond to the therapy and/or administering a treatment that does not comprise the therapy to a subject who has been determined as unlikely to respond to the therapy.

Also described herein according to a fourth aspect is a method of determining whether a subject with cancer is likely to develop a gene amplification, the method comprising: characterising a DNA sample obtained from a tumour of the subject, using the method of any embodiment of the first aspect, as having at least one segment with a metric associated with one or more predetermined signatures of chromosomal instability satisfying a predetermined criterion and/or as having a sample exposure to said signatures satisfying a predetermined criterion; wherein the presence of at least one segment with a metric associated with the one or more predetermined signatures of chromosomal instability satisfying a predetermined criterion and/or as having a sample exposure to said signatures satisfying a predetermined criterion indicates that the subject is likely to develop an amplification of a gene. The gene may be an oncogene.

Also described herein according to a fifth aspect is a method of providing a prognosis for a subject with cancer, the method comprising: determining whether the is likely to develop a gene amplification using the method of the fourth aspect; and determining a prognosis for said subject, wherein a subject identified as likely to develop an amplification of the gene has poorer prognosis than a subject for which a sample is characterised as not having at least one segment with the metric associated with the one or more signatures satisfying a predetermined criterion and/or not having a sample exposure to said signatures satisfying a predetermined criterion.

Also described herein according to a sixth aspect is a method of providing a prognosis for a subject with cancer, the method comprising: characterising a DNA sample obtained from a tumour of the subject, using the method of any embodiment of the first aspect, as having at least one segment with a metric associated with one or more predetermined signatures of chromosomal instability satisfying a predetermined criterion and/or as having a sample exposure to said signatures satisfying a predetermined criterion; wherein the presence of at least one segment with a metric associated with the one or more predetermined signatures of chromosomal instability satisfying a predetermined criterion and/or as having a sample exposure to said signatures satisfying a predetermined criterion indicates that the subject has poorer prognosis than a subject for which a sample is characterised as not having at least one segment with the metric associated with the one or more signatures satisfying a predetermined criterion and/or not having a sample exposure to said signatures satisfying a predetermined criterion.

Also described herein according to a seventh aspect is a method of determining whether a particular process causing chromosomal instability is currently active in a subject's cancer, the method comprising: characterising a sample obtained from said subject using the method of any embodiment of the first aspect, wherein the tumour copy number profiles are obtained from single cell sequencing data and the characterising is performed individually using each of a plurality of single cell tumour copy number profiles; identifying one or more shared copy number events as events that are present in a proportion of single cell tumour copy number profiles above a predetermined threshold and/or one or more unique copy number events that are present in a proportion of single cell tumour copy number profiles at or below the predetermined threshold; wherein a process causing chromosomal instability associated with a signature that has been determined to be associated with a shared copy number event is likely to have been active in the tumour prior to at least one clonal expansion in the tumour, and/or a process causing chromosomal instability associated with a signature that has been determined to be associated with a unique copy number event is likely to be currently active in the tumour.

According to a further aspect, there is provided one or more non-transitory computer readable media comprising instructions that, when executed by one or more processors, cause the one or more processors to perform the steps of any method described herein, such as a method according to any embodiment of any preceding aspect.

According to a further aspect, there is provided a computer program comprising code which, when the code is executed on a computer, causes the computer to perform the steps of any method described herein, such as a method according to any embodiment of any of the first to seventh aspects.

According to a further aspect, there is provided system comprising: a processor; and a computer readable medium comprising instructions that, when executed by the processor, cause the processor to perform the steps of any method described herein, such as a method according to any embodiment of any of the first to seventh aspects.

BRIEF DESCRIPTION OF THE FIGURES

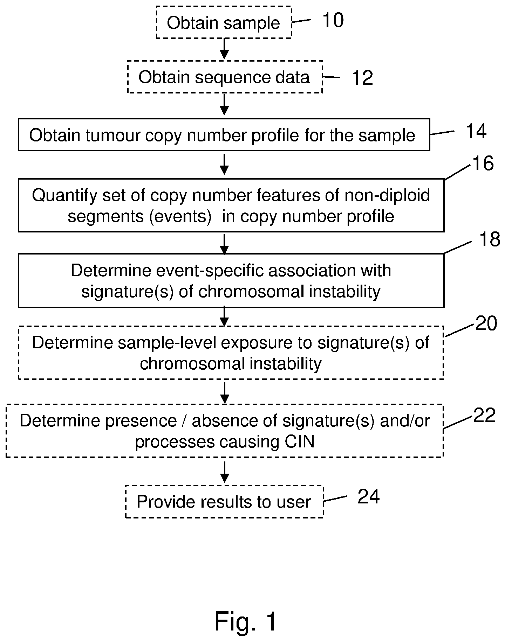

Figure 1 is a flowchart illustrating schematically a method of identifying one or more copy number signatures associated with an individual copy number alteration event in a sample.

Figure 2 is a flowchart illustrating schematically methods of characterising a tumour's evolutionary history and/or heterogeneity, providing a prognosis, identifying a therapy or treating a subject.

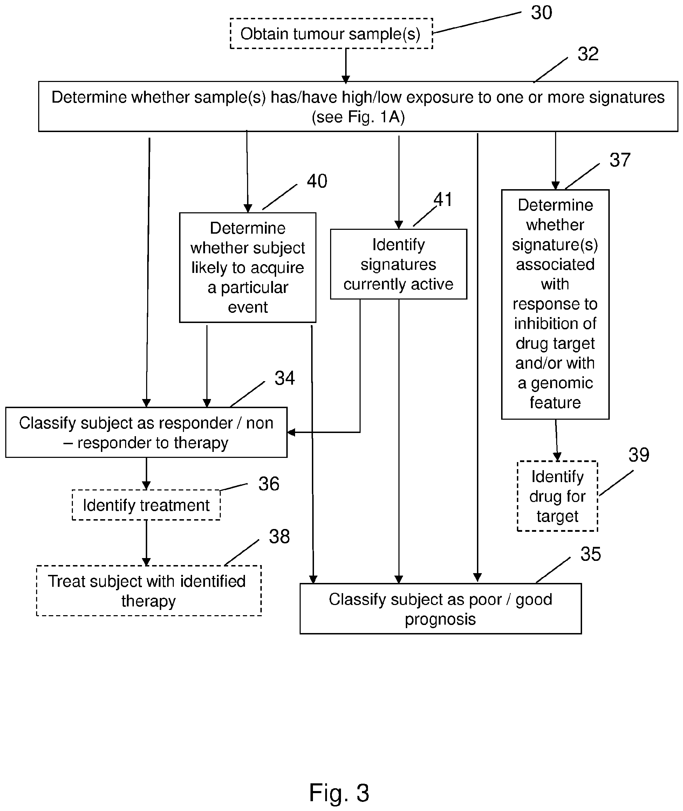

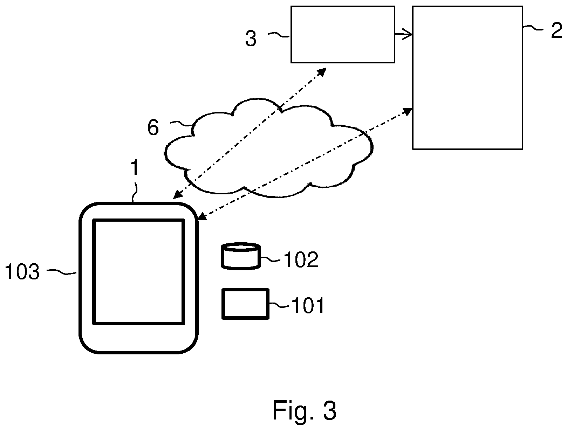

Figure 3 shows an embodiment of a system for performing methods of the disclosure.

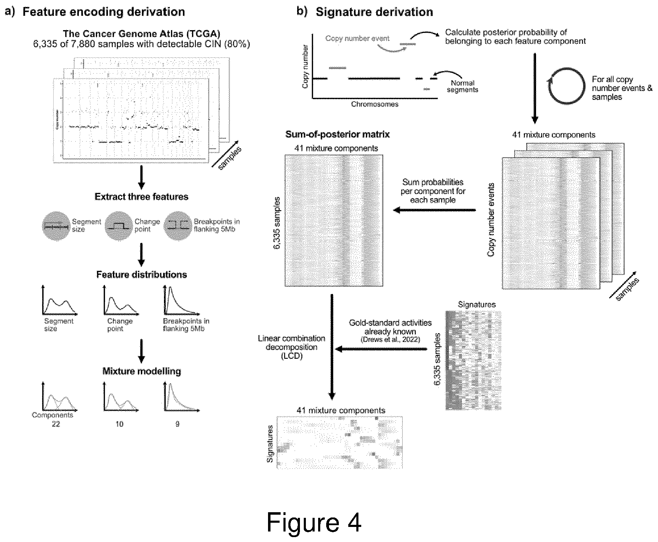

Figure 4 shows a schematic overview of copy number features and CIN signature identification according to embodiments of the disclosure, a) Schematic of feature encoding design and derivation of feature components. Mixture modelling was applied to split the 3 feature distributions into smaller components by either Variational Bayes Gaussian mixture models (for segment size and change point) or Finite Poisson mixture models (for breakpoints in flanking 5Mb). b) For all samples and features, the probability of each copy number event (no diploid segments) to belong to each mixture component is computed. Then, component probabilities of all events are summed to obtain a 41-dimensional feature vector per sample. In the illustrated examples, the activities of CIN signatures according to Drews et al. 2022 were already known for all 6,335 samples of the TCGA cohort (see Example 2 below). Therefore, linear combination decomposition was applied for deriving signature definitions in the new feature space.

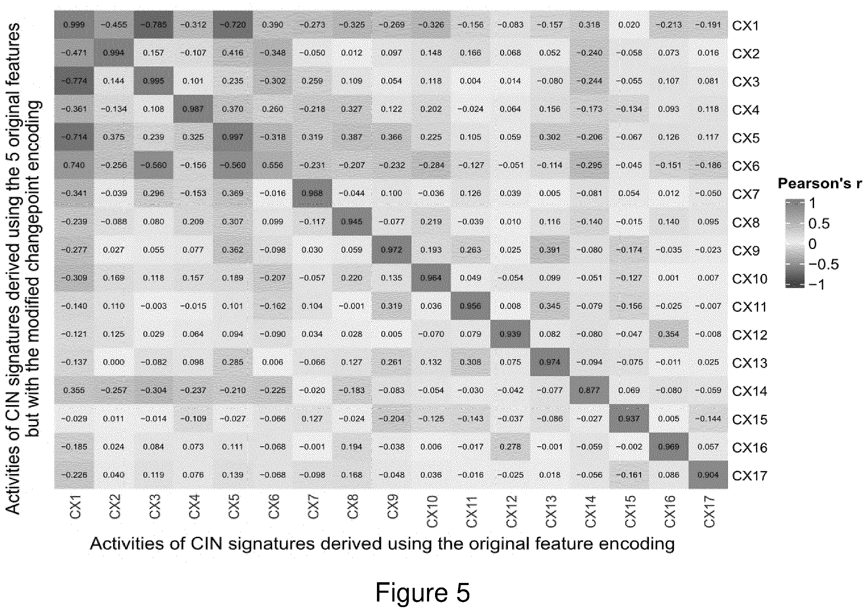

Figure 5 shows data investigating the impact of modifying the changepoint feature encoding in CIN signatures. The heatmap shows Pearson correlation coefficients between CIN signature activities derived by using the feature encoding (x-axis) described in Drews et al. 2022 and those derived by extracting the 5 original features but using the modified encoding version of the changepoint feature described in Example 1. Red colour denotes a high positive correlation between activities, while blue colour denotes a high negative correlation.

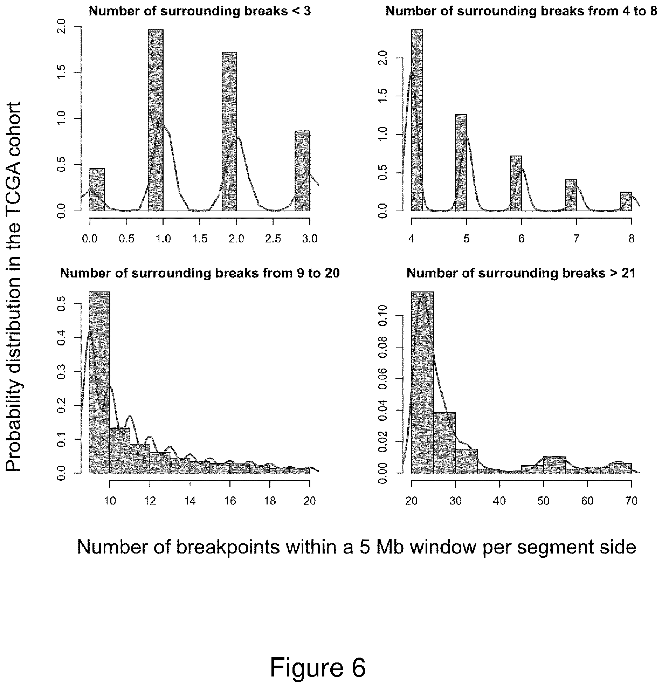

**Figure 6** displays histograms and fitted density curves showing the distribution of the breakpoints per side feature in the TCGA cohort (see Example 1). For visualisation purposes, the data are split in four subsets according to the number of breakpoints within a 5 Mb window per segment side. The combined distribution is used for deriving the different feature components capturing the signal of this novel featureI.

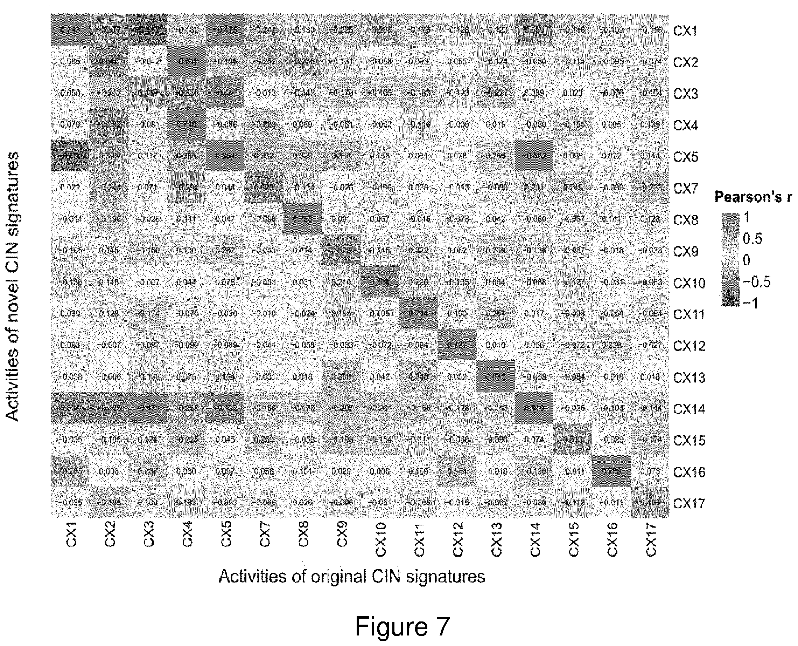

**Figure 7** shows a comparison of CIN signatures encoded using the original features (Drews et al. 2022) and the new features of the present disclosure. The heatmap shows Pearson's r correlation coefficients between activities of original (x-axis) and novel (y-axis) CIN signatures computed in 6,335 TCGA samples. Red colour denotes a high positive correlation between activities, while blue colour denotes a high negative correlation.

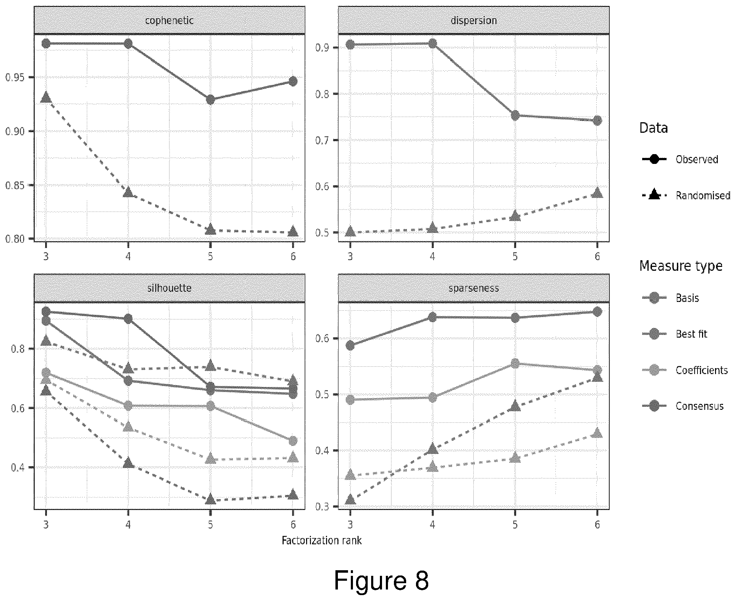

**Figure 8** shows results of measures used for selecting the optimal signature number in simulated genomes. A comparison of four evaluation measures obtained for each signature number (x axis). The circle and solid lines represent the results from the simulated dataset, whereas the triangles and dotted lines represent results from 1,000 randomly permuted input matrices (these can be considered a null measure). Here basis refers to the signature definition matrix, coefficients refers to the activity matrix (exposure), and consensus refers to the connectivity matrix of samples clustered by their dominant signature across 1,000 runs. A value of 5 defines the point of stability in the cophenetic, dispersion and silhouette coefficients and is the maximum sparsity achievable above the null model for the basis matrix.

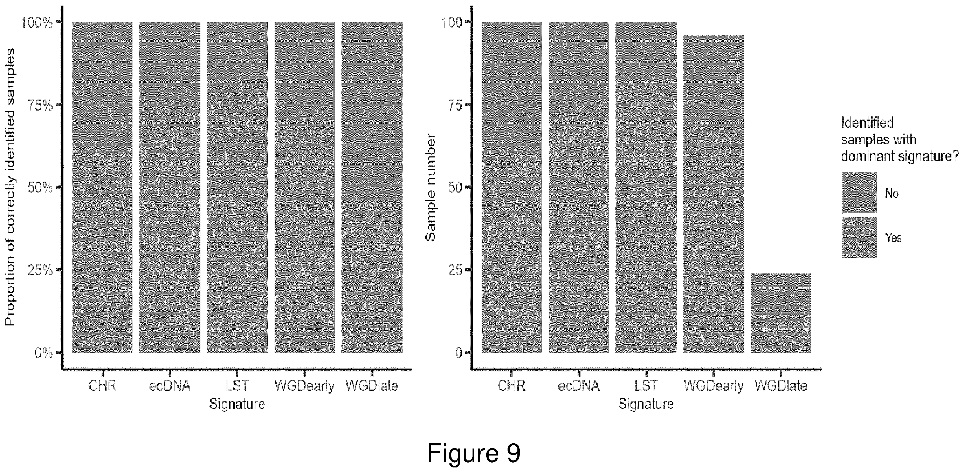

**Figure 9** shows data demonstrating the ability of the methods of the disclosure to capture simulated CIN types. Barplot showing the accuracy of signature activities identifying samples with a dominant mutational process as produced by simulation parameters. Proportion (left) and absolute number (right) of correctly identified samples for each mutational process.

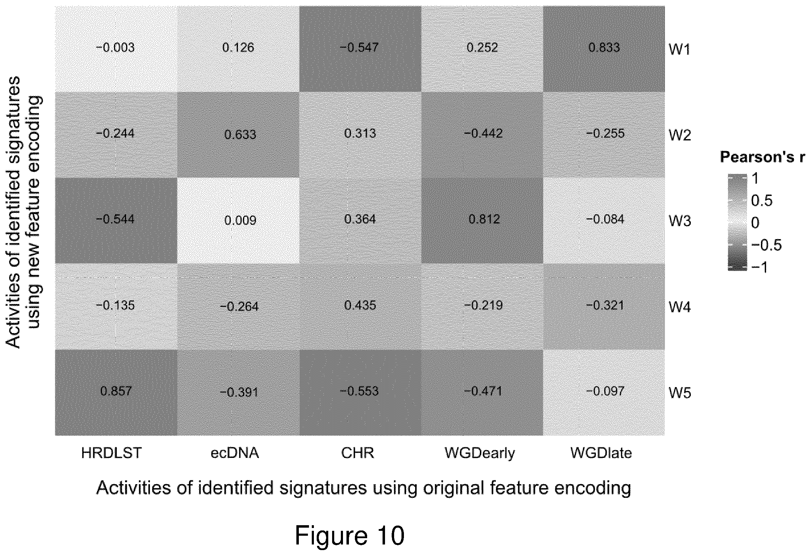

**Figure 10** shows a comparison of signatures extracted from simulated genomes using the original (Drews et al. 2022) and new feature encoding (present disclosure). The heatmap shows Pearson's r correlation coefficients between activities of signatures derived from a simulated dataset using the original (x-axis) and novel (y-axis) feature encoding. Red colour denotes a high positive correlation between activities, while blue colour denotes a high negative correlation.

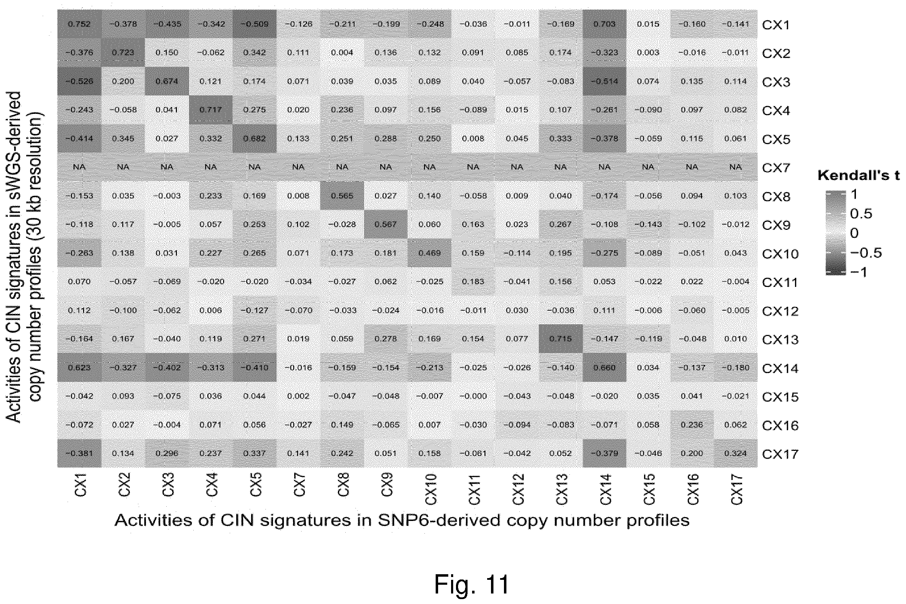

**Figure 11** shows data investigating CIN signature stability across different copy number profiling technologies (signatures defined according to the present disclosure). The heatmap shows Kendall's tau correlation coefficients between CIN signature activities derived from SNP6 (x-axis) and sWGS (y-axis) data from the same set of 478 tumours. Red colour denotes a high positive correlation between activities, while blue colour denotes a high negative correlation.

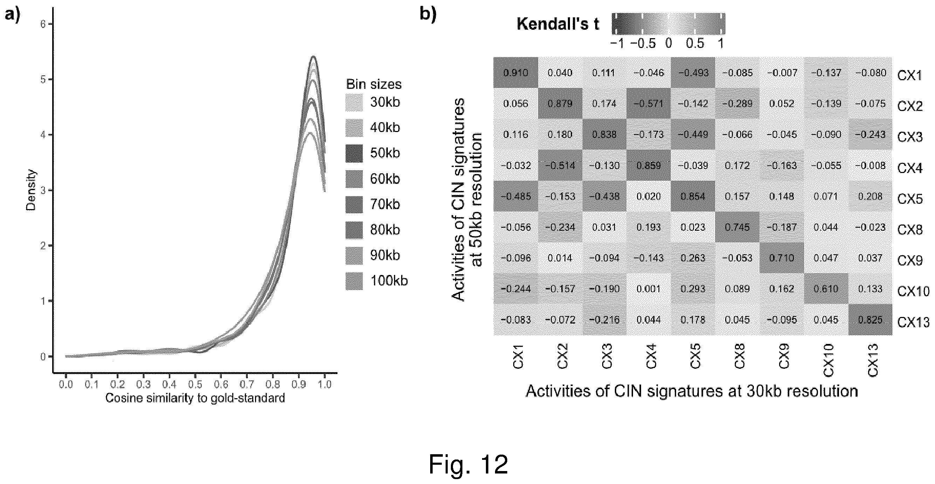

**Figure 12** shows data investigating CIN signature stability across different copy number profiling resolutions (signatures defined according to the present disclosure). a) Density plot showing cosine similarities of CIN signature exposures in SNP6-derived profiles with those obtained from 478 sWGS-derived profiles using different bin resolutions. b) Heatmap showing Kendall's tau correlation coefficients between CIN signature exposures derived from sWGS data segmented using a bin size of 30 kb (x-axis) and 50 kb (y-axis). The same set of 478 tumours was used for comparisons. Red colour denotes a high positive correlation between exposures, while blue colour denotes a high negative correlation.

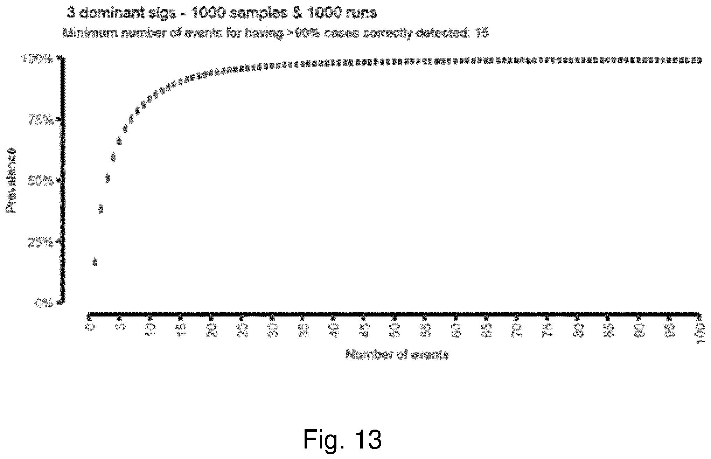

**Figure 13** shows data investigating the number of individual events required for sample-level signature quantification, using sample signature composition define by the three dominant signatures for each sample.

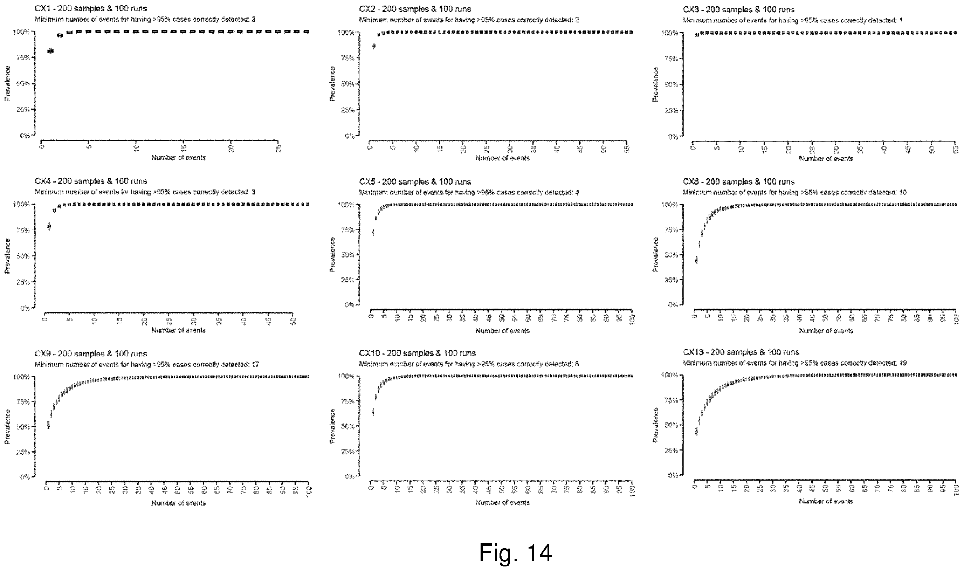

**Figure 14** shows data investigating the number of individual events required for sample-level signature quantification, for individual signatures.

DETAILED DESCRIPTION

In the present disclosure, the following terms will be employed, and are intended to be defined as indicated below.
The present disclosure relates broadly to the characterisation of DNA samples, particularly tumour samples, in terms of their copy number profiles.

A "copy number profile" refers to the quantification of the number of copies for each of a plurality of portions of a genomic sequence. In the context of the present disclosure, a copy number profile is preferably a genome-wide copy number profile. A copy number profile is obtained by analysing sequencing data. For example, a copy number profile may be obtained by obtaining sequence data from a sample of genomic DNA (or a DNA library derived therefrom, as explained further below, including a sample of DNA derived from genomic DNA by fragmentation, such as e.g. cell free DNA), and quantifying the number of copies per portion (e.g. per bin, where a bin can be e.g. a 30kb region) of the genomic sequence, as known in the art. A "tumour copy number profile" refers to a copy number profile that is associated with a tumour genome. A tumour copy number profile may be obtained by sequencing a sample comprising tumour genomic DNA, as explained further below. In embodiments, a tumour copy number profile is a copy number profile that has been obtained by sequencing a sample of genomic DNA derived from tumour cells. Thus, a copy number profile is typically in the form of an estimate, for each of a plurality of genomic segments, of the number of copies of the segment in the sample or a subset of the sample (e.g. in the case of samples comprising cells with different genotypes or genetic material derived therefrom, such as e.g. samples comprising a mixture of tumour and normal cells or tumour and normal DNA). Copy number profiles can be obtained by segmentation and copy number estimation from read count data using methods known in the art. For example, methods such as circular binary segmentation may be used for segmentation. Copy number fitting may be performed as described in the examples below. Alternatively, methods such as Ascat (Raine et al. 2016), ichorCNA (Adalsteinsson et al. 2017) and others may be used to perform segmentation and copy number estimation (and also % tumour DNA estimation, in the case of Ascat and ichorCNA). A copy number profile may be a genome-wide copy number profile. A genome-wide copy number profile may be obtained from a sample using WGS, sWGS or single cell WGS.

A "segment" in a copy number profile refers to a portion of a sequence represented in a copy number profile which is associated with a consistent absolute copy number. The consistent copy number is different from that associated with the sequence directly upstream (if such a sequence is present and associated with a copy number estimate) and the sequence directly downstream (if such a sequence is present and associated with a copy number) of said portion. In other words, a segment refers to a portion of sequence that has a copy number associated with it, where the copy number associated with the segment differs from the copy number associated with its immediate neighbouring segment(s). The copy number associated with a segment may differ from the copy number associated with its immediate neighbouring segment(s) because the segment(s) that surround the segment are associated with a different copy number, because the segment(s) that surround the segment are not associated with a copy number (e.g. because data for the segment(s) is missing, or of insufficient quality), or a combination of both (e.g. a segment may be surrounded by a segment that is associated with a different copy number on one side, and a segment that is not associated with a copy number on the other side). In other words, segments refer to the longest continuous portion of a copy number profile that are each associated with a single copy number. A segment may be associated with a set of coordinates, e.g. genomic coordinates, which define the boundaries of the segment. Each boundary may be associated with a copy number changepoint (also referred to as "changepoint"). In embodiments, the copy number profiles comprise at most 350 segments (copy number events), at most 300 segments, or at most 250 segments. Preferably, the copy number profiles comprise at most 250 segments (copy number events). These numbers may be particularly useful when looking at human genome wide copy number profiles. Without wishing to be bound by theory, it is believed that higher numbers of segments may be indicative of unwanted DNA degradation (such as e.g. formalin-mediated DNA degradation). The copy number of a segment may in practice be a copy number estimate obtained using a method for determining tumour copy number profiles, such as e.g. ASCAT [Van loo et al.,2010] or Sequenza [Favero et al., 2015]. According to the present disclosure a "copy number event" refers to a single segment in a copy number profile. In particular, a copy number event may refer to a segment with a copy number that deviates from the expected copy number in a normal genome (e.g. a non-diploid segment). Copy number events / segments are characterised by a plurality of copy number features, further defined below. Thus, the terms "copy number event" and "segment" may be used interchangeably. Note that this is not necessarily the case in the prior art, such as e.g. in WO 2023/057392 where copy number features are determined for copy number events that encompass multiple segments. In the present disclosure, all features are determined in relation to a specific copy number event / segment.

Chromosomal instability (CIN) is the process of accumulating numerical and structural changes in DNA. A signature of chromosomal instability (CIN) (also referred to herein as copy number signature or "signature") is a signature (typically in the form of a set of weights associated with respective categories of copy number features, also referred to as "components") representing the genome-wide imprint of distinct putative mutational processes (where a "mutational process" as used herein refers to any process that can cause chromosomal instability). CIN signatures are described in Macintyre et al. 2018, Drews et al. (2022) and in WO 2023/057392, which are incorporated herein by reference. The presence or absence of a CIN signature in a sample may be determined by calculating the exposure (also referred to as "activity") of the sample to the signature. A "CIN signature" is a set of weights associated with each of a plurality of components. The plurality of weights may sum to 1 and may each represent the probability that a mutational process associated with the signature generates a copy number event with a particular characteristic defined by the component. In other words, the plurality of weights of a signature may each represent the probably that a mutational process associated with the signature generates a particular type of copy number event. Each type of copy number event is referred to as a "component". Each component is defined by a predetermined distribution for possible values of a copy number feature.

The term "copy number (CN) feature" refers to properties of copy number events observable in a copy number profile. According to the present disclosure, copy number features include: segment size (also referred to as "segment length" typically expressed in number of bases), change-point copy number (the absolute difference in copy number between a segment and an adjacent/neighbouring segment in the copy number profile, which may be defined relative to the upstream segment, the downstream neighbouring segment, or a referencecopy number state, typically 2 when analysing normally diploid genomes), and number of breakpoints per flanking region (also referred to as "breakpoints per side", the number of breakpoints, i.e. changes in copy number / junction between individual segments in a flanking region of the segment, which can be upstream or downstream of the segment). The flanking region used to determine the "breakpoints per side" feature may have a predetermined length between 2 and 10 Megabases (Mb), preferably between 3 and 8Mb, or about 5 Mb. The number of breakpoints per flanking region may be determined for one or both flanking regions, and a summarised metric may be obtained when both flanking regions are used. The summarised metric may be the maximum or average number of breakpoints observed in the upstream and downstream flanking regions. In embodiments, the breakpoint per side feature for a segment is the maximum number of breakpoints observed in the upstream or downstream flanking region of the segment. The copy number features according to the present disclosure do not include any feature that is not uniquely associated with individual events. In other words, the copy number features according to the present disclosure do not include any copy number feature that is quantified over a genomic region that is not defined by reference to a segment. In particular, the copy number features according to the present disclosure do not use any of: breakpoint count per x MB (the number of changepoints appearing in a sliding windows across the copy number profile), breakpoint count per chromosome arm (the number of changepoints occurring per chromosome arm), and number of segments with oscillating copy number (sometimes referred to as "length of segments with oscillating copy number"; number of continuous segments alternating between two copy number states, rounded to the nearest integer copy-number state; also referred to as length of chain of oscillating copy number states). In embodiments, the copy number features used according to the present invention do not include the segment copy number (the observed absolute copy number state of each segment, also referred to herein as "copy number" or "absolute copy number"). Each CN feature is associated with one or more components. Each component is defined by a distribution. CN features are observed for individual copy number events. Typically, CN features are observed for each event on a genome-wide basis for a sample or collection of samples, and summarised metrics are obtained across events in a sample for each component. These summarised metrics are in turn used to determine signature exposures for the sample. According to the present disclosure, signature exposures are determined for individual events, and optionally also for a sample (i.e. for a whole copy number profile).

Methods for determining the exposure to a signature, from a copy number profile (i.e. at a sample level), as well as identifying signatures from a cohort of samples are known in the art (see e.g. Macintyre et al., 2018, Drews et al. (2022)). In particular, the determination of the exposure of a sample to one or more CN signatures (i.e. activity of the one or more signatures in the sample) may be performed by identifying the matrix $E$ that satisfies $C \approx PE$ where C is a mutational catalogue for one or more samples for which exposure is to be determined (also referred to as patient-by-component, PbC matrix), P is a signature matrix comprising the one or more CN signatures for which exposure is to be determined (also referred to as signature-by-component, SbC matrix), and E is an exposure matrix (also referred to as patient-by-signature, PbS). For example, exposure to a signature may be determined by performing matrix decomposition to identify the value (or vector of values) that satisfies the equation: PbC ≈ PbS x SbC (or, in practice PbC = PbS x SbC + ε, where ε is a residual term to be minimised), where SbC is the row of the signature-by-component matrix that corresponds to a particular signature of a set of signatures, and PbS is the patient-by-signature value (or vector of values). Exposure to multiple copy number signatures can be calculated using the corresponding rows of the signature-by-component matrix. In embodiments, exposure (E) to a copy number signature *i* as described herein (from a set of *n* signatures, where n can be e.g. 16 or 17, where the signatures can include any or all of the signatures disclosed herein or corresponding signatures) is the value $E_i$ that satisfies the equation:

$$PbC \approx E \ x \ SbC \qquad \text{(Equation 1)}$$

where: E is a vector of size n comprising coefficients $E_{1,...,n}$ where $E_i$ is the exposure to signature *i*; PbC is a vector of size c, each value representing the sum-of-posterior probabilities of each copy number event in the copy number profile belonging to a component C, where each component C is a distribution of values for a copy number feature; and SbC is a matrix of size c by n, each value representing the weight of a component C in a copy number signature i. This is equivalent to solving the minimisation problem $\min(\|E*SbC-PbC\|)$ with constraints on nonnegativity of E, with *SbC* and *PbC* being known. For example, linear combination decomposition as implemented in R package YAPSA (Hübschmann, D. et al. 2021) may be used for this purpose. A similar approach can be used to identify signatures using a cohort of samples and nonnegative matrix factorisation (see e.g. Drews et al. 2022) to identify both an exposure matrix *E* and a signature matrix *SbC.* The present disclosure provides, in addition to the determination of exposure of samples to signatures, the determination of associations between signatures and individual segments (in particular, copy number events) in a copy number profile. This will be explained further below.

In the context of the present disclosure, each component represents a type of copy number abnormality defined by an individual component of a mixture model fitted to the distribution of values obtained for each of 3 copy number features described herein over a cohort of samples from the Cancer Genome Atlas (TCGA). Note that any other cohort of cancer samples can be used for this purpose and the disclosure is not limited in any way in relation to the cohort of samples from which the components (and corresponding signatures) are extracted. This follows a process as described in Drews et al. 2022, except that three features in Drews et al. 2022 (breakpoints per chromosome arm, breakpoints per 10 Mb, number of segments with oscillating copy number) were removed and replaced by a new feature as described herein (breakpoints per side). The components in Drews et a. 2022 were used for the segment size and changepoint features, and new components were identified for the new feature. Copy number signatures (i.e. signatures of copy number alteration processes) are obtained using these, which capture the probability of copy number alteration processes causing copy number events that are distributed according to each of the copy number feature components. Thus, the term "exposure" (also referred to as "signature activity" or "activity") in this context captures the strength of evidence for the presence of copy number alteration events attributable to the signature.

The term "genome-wide" refers to data that covers a whole genome or substantial parts thereof such as e.g. one or more entire chromosomes. As the skilled person understand, sequencing data or a copy number profile derived therefrom may be referred to as "genome wide" even though it may not contain information (i.e. reads or a copy number estimate) for each and every single position in the genome. Indeed, even in the context of sWGS there may be regions of the genome that have no mapping reads due to e.g. low mappability, sequencing difficulty, or the sampling nature of next generation sequencing. Thus, data may be referred to as "genome-wide" if it contains data for at least 70%, at least 80% or at least 90%, preferably at least 90%, of one or more chromosomes or all chromosomes present in a reference genome.

A "sample" as used herein may be a cell or tissue sample, a biological fluid, an extract (e.g. a DNA extract obtained from a cell, tissue or fluid sample), from which genomic material can be obtained for genomic analysis, such as genomic sequencing (e.g. whole genome sequencing, or targeted sequencing such as whole exome sequencing or targeted panel sequencing). The sample may be a cell, tissue or biological fluid sample obtained from a subject (e.g. a biopsy). Such samples may be referred to as "subject samples". The sample may be any sample comprising genomic DNA or cell free DNA. In particular, the sample may be a tumour sample, a biological fluid sample containing DNA or cells, a blood sample (including plasma or serum sample), a urine sample, a cervical smear, an ascites fluid sample, or a sample derived therefrom (e.g. after DNA purification). It has been found that urine, ascites fluid and cervical smears contains cells, and so may provide a suitable sample for use in accordance with the present invention. Other sample types suitable for use in accordance with the present invention include fine needle aspirates, lymph nodes samples (e.g. aspirates or biopsies), surgical margins, bone marrow or other tissue from a tumour microenvironment, where traces of tumour DNA may be found or expected to be found. The sample may be one which has been freshly obtained from a subject or may be one which has been processed and/or stored prior to genomic/transcriptomic analysis (e.g. frozen, fixed or subjected to one or more purification, enrichment or extraction steps). The sample may be a cell or tissue culture sample. As such, a sample as described herein may refer to any type of sample comprising cells or genomic material derived therefrom, whether from a biological sample obtained from a subject, or from a sample obtained from e.g. a cell line. In embodiments, the sample is a sample obtained from a subject, such as a human subject. The sample is preferably from a mammalian (such as e.g. a mammalian cell sample or a sample from a mammalian subject, such as a cat, dog, horse, donkey, sheep, pig, goat, cow, mouse, rat, rabbit or guinea pig), preferably from a human (such as e.g. a human cell sample or a sample from a human subject). Further, the sample may be transported and/or stored, and collection may take place at a location remote from the sequence data acquisition (e.g. sequencing) location, and/or any computer-implemented method steps described herein may take place at a location remote from the sample collection location and/or remote from the sequence data acquisition (e.g. sequencing) location (e.g. the computer-implemented method steps may be performed by means of a networked computer, such as by means of a "cloud" provider). A sample may be a tissue biopsy, such as a sample of fresh frozen tissue or a sample of formalin-fixed paraffin embedded (FFPE) tissue, or a sample comprising circulating tumor DNA (ctDNA) such as e.g. a sample of biological fluid (sometimes referred to as liquid biopsy). The samples used in methods of the present disclosure are typically samples comprising tumour cells (e.g. a tumour sample or sample comprising circulating tumour cells) or genetic material derived from tumour cells (such as e.g. cell free DNA, or DNA extracted from a sample comprising tumour cells - e.g. from a cell line or tissue sample - or circulating tumour cells). A sample may be a "mixed" sample comprising cells with different genotypes or genetic material derived therefrom. For example, a sample may be a sample comprising tumour cells and normal cells, or DNA derived therefrom, such as e.g. in the context of cell free DNA (cfDNA) samples comprising circulating tumour DNA (ctDNA). Copy number profiles may be determined separately for cells that have a genotype comprising somatic copy number alterations, in a sample comprising both cells that have the genotype comprising somatic copy number alterations (e.g. tumour cells) and cells that do not contain such alterations (e.g. normal cells), using methods known in the art. For example, sequence analysis processes that deconvolute tumour and germline genomes from copy number profiles include ASCAT (Raine et al., 2016), ABSOLUTE (Carter et al., 2012), or, in the context of cfDNA, ichorCNA (Adalsteinsson et al., 2017). A "tumour sample" refers to a sample that contains tumour cells or genetic material derived therefrom. The tumour sample may be a cell or tissue sample (e.g. a biopsy) obtained directly from a tumour. A tumour sample may be a sample that comprises tumour cell or genetic material derived therefrom, that has not be obtained directly from a tumour. For example, a tumour sample may be a sample comprising circulating tumour cells or circulating tumour DNA. Thus, a tumour sample may also be a biological fluid (e.g. a liquid biopsy such as a blood, urine, or cerebrospinal fluid biopsy). A sample comprising a mixture of tumour cells and other cells (or material genetic derived therefrom) may be subject to one or more processing steps, whether prior to or subsequent to the acquisition of sequence data, in order to identify sequence data that is representative of the genetic material from the tumour. For example, a sample comprising cells may be subject to one or more cell purification steps which selectively enrich the sample for tumour cells. As another example, a sample of genetic material may be subject to one or more capture and/or size selection steps to selectively enrich the sample for tumour-derived genetic material. Protocols for doing this are known in the art. As another example, sequence data may be subject to one or more filtering steps (e.g. based on fragment length in the context of cell-free DNA samples) to enrich the data for information that relates to tumour-derived genetic material. Protocols for doing this are known in the art. In embodiments, the sample is a sample comprising tumour cells. Preferably, such a sample has a tumour purity (where tumour purity can be quantified as the proportion of cells in the sample that are tumour cells) of at least 30%, at least 35%, at least 40%, at least 45%, or at least 50%. Advantageously, the sample has a tumour purity of at least 40%. Without wishing to be bound by theory, it is believed that the copy number profiles generated from samples that have lower tumour purity may be less suitable as the signal corresponding to the tumour genome may be lost amongst the signal from the genomes of other cells. A "normal sample" (also referred to as "germline sample") refers to a sample that contains non-tumour or non-modified cells or genetic material derived therefrom. A normal sample may be matched to a particular tumour or modified sample in the sense that it is obtained from the same biological source (subject or cell line) as the tumour or modified sample. A matched normal sample is a sample that is expected to be representative of the genome of a tumour sample in the absence of somatic abnormalities. This is typically a sample obtained from the same subject as the tumour sample. A normal sample may be used in the context of the present invention as a control to analyse a tumour sample. For example, a tumour sample and a (typically matched) normal sample may be analysed together in order to obtain a copy number profile for the tumour. Methods to obtain tumour copy number profile from a pair of normal and tumour samples are known in the art. For example, these include ASCAT [Van Loo et al. 2010] and Sequenza [Favero et al. 2015]. Such methods may provide a tumour copy number profile as well as an estimate of the purity (proportion of tumour cells) of the tumour sample. The purity estimate may be used to exclude tumour samples that have low purity and hence could lead to low quality copy number estimates.

The term "sequence data" refers to information that is indicative of the presence of genetic material in a sample that has a particular sequence. Such information may be obtained using sequencing technologies, such as e.g. next generation sequencing (NGS), for example whole exome sequencing (WES), whole genome sequencing (WGS), or sequencing of captured genomic loci (targeted or panel sequencing), or using array technologies, such as e.g. SNP arrays, or other molecular counting assays. In embodiments, the sequence data is obtained by DNA sequencing, and particularly next generation sequencing. In such embodiments, the sequence data comprises sequencing reads, or information derived therefrom such as the count of the number of sequencing reads that have a particular sequence. When non-digital technologies are used such as array technology, the sequence data may comprise a signal (e.g. an intensity value) that is indicative of the number of sequences in the sample that have a particular sequence, for example by comparison to an appropriate control. Information such as the count of sequencing reads that have a particular sequence can be derived from sequencing reads by mapping the sequence data to a reference sequence, for example a reference genome, using methods known in the art (such as e.g. Bowtie (Langmead et al., 2009) or BWA (Li and Durbin 2009)). This may result in aligned sequencing reads, for example in the form of a SAM or BAM file. Thus, sequence data may be associated with a particular genomic location (where the "genomic location" refers to a location in the reference genome to which the sequence data was mapped). Sequence data may be filtered as part of the methods described herein or may have been filtered prior to application of the methods described herein, for example to remove low quality reads or remove reads that map to regions that have "low mappability". Methods for filtering low quality reads are known in the art, and include e.g. removing duplicate reads, removing supplementary alignments, removing reads with low sequencing quality scores (reads below Q20 or Q30), removing reads with low mapping quality score (e.g. MAPQ<37), etc. Methods to filter reads that map to regions that have low mappability are known in the art and include e.g. Umap (Karimzadeh et al. 2018), and GenMap (Pockrandt et al. 2020).

A composition as described herein may be a pharmaceutical composition which additionally comprises a pharmaceutically acceptable carrier, diluent or excipient. The pharmaceutical composition may optionally comprise one or more further pharmaceutically active polypeptides and/or compounds. Such a formulation may, for example, be in a form suitable for intravenous infusion.

As used herein "treatment" refers to reducing, alleviating or eliminating one or more symptoms of the disease which is being treated, relative to the symptoms prior to treatment.

"Patient" as used herein in accordance with any aspect of the present invention is intended to be equivalent to "subject" and specifically includes both healthy individuals and individuals having a disease or disorder (e.g. a proliferative disorder such as a cancer). Preferably, the patient is a human patient. In some cases, the patient is a human patient who has been diagnosed with, is suspected of having or has been classified as at risk of developing, a cancer.

As used herein, the terms "computer system" includes the hardware, software and data storage devices for embodying a system or carrying out a method according to the above described embodiments. For example, a computer system may comprise a central processing unit (CPU) and/or a graphic processing unit (GPU), input means, output means and data storage, which may be embodied as one or more connected computing devices. A computer system may comprise a display or comprises a computing device that has a display to provide a visual output display. The data storage may comprise RAM, disk drives or other non-transitory computer readable media. The computer system may include a plurality of computing devices connected by a network and able to communicate with each other over that network. It is explicitly envisaged that computer system may consist of or comprise a cloud computer.

As used herein, the term "computer readable media" includes, without limitation, any non-transitory medium or media which can be read and accessed directly by a computer or computer system.

The media can include, but are not limited to, magnetic storage media such as floppy discs, hard disc storage media and magnetic tape; optical storage media such as optical discs or CD-ROMs; electrical storage media such as memory, including RAM, ROM and flash memory; and hybrids and combinations of the above such as magnetic/optical storage media.

Chromosomal instability signatures at the level of single copy number events

The present disclosure provides methods for characterising a DNA sample by identifying signatures of chromosomal instability associated with individual copy number alterations in a sample.

**Figure 1** is a flow diagram showing, in schematic form, a method of characterising a DNA sample according to the disclosure. At optional step 10, a DNA sample is obtained from a tumour of a subject. Optionally, a matched normal sample may also be obtained from the subject. At optional step 12, sequence data is obtained from the tumour (and optionally the matched normal) DNA sample(s), for example using sequencing or array technologies. At step 14, a tumour copy number profile for the sample is obtained, for example by analysing the sequence data obtained at step 12 or by receiving the copy number profile from a user, database etc. Thus, steps 10-12 are optional because methods described herein may start from a previously obtained tumor copy number profile for the sample. The data may be bulk sequence data (from a bulk DNA sequencing technology or an array technology) or single cell sequencing data (from a single cell DNA sequencing technology). When single cell sequencing data is used, the process described by reference to steps 12-26 may be performed individually for each of a plurality of single cells. Step 14 of obtaining a tumour CN profile may comprise one or more of: aligning a plurality of sequence reads to a reference genome, determining that the copy number profile comprises a number of non-diploid segments above a predetermined threshold (e.g. at least 15 or at least 20 events in the copy number profile), determining a copy number for each of a plurality of genomic bins of a predetermined size, excluding genomic bins comprising a number of mapped reads below a predetermined threshold, and/or performing absolute copy number fitting. The predetermined bin size may be at most 100kb, at most 50kb, or about 30kb. The predetermined threshold on number of mapped reads may be 15 reads.

At step 16, a set of copy number features are quantified, for each of one or more segments in the copy number profile. The set of copy number features consists of copy number features that are associated with respective segments. In other words, the set of copy number features may not include any copy number feature that is defined for a section of the genome that is not a segment or a region defined by reference to a segment. As such, each copy number feature is defined for a specific segment, and quantifies a property of the segment and/or a region defined by reference to a segment (such as e.g. a flanking region of a predetermined length, a region of a predetermined length centred on the segment, etc.).

Step 16 comprises quantifying the set of copy number features for each segment. This step may use unrounded copy number segments. This step may comprise collapsing and merging near diploid segments to a diploid state, wherein near diploid segments are segments that have a copy number within a predetermined distance from 2. The predetermined distance may be 0.1. Thus, quantifying the set of copy number features may comprise assigning a copy number of 2 (i.e. collapsing) to any segment that has a copy number within predetermined boundaries (such as e.g. above 1.9 and below 2.1), and merging any contiguous segments that have the same copy number as a result of the assigning. This may advantageously avoid including signal from segments that are likely normal diploid segment and which could act as noise in the process. Unrounded copy number segments may be segments whose copy number has not been rounded to the near integer. Thus, the method preferably uses copy number profiles that have not been rounded and/or wherein the only rounding that is performed relates to the collapsing or near diploid segments. This uses more of the information present in copy number profiles (in terms of number of segments that would otherwise be merged but also in terms of the copy number information that is contained in said profile for each segment). Rounding of copy numbers for segments may be performed in order to remove noise in a copy number profile, at the cost of loss of information. It has been previously shown that the additional noise that is associated with the use of unrounded copy number segments could be compensated at least in part, with minimal loss of information relevant to CIN by merging segments with minor deviations from the "normal" copy number state, and the present inventors have shown that this also applies to the new methods described herein.

The set of copy number features quantified for each segment at step 16 may comprise a segment size feature, a copy number changepoint feature, and a breakpoint per side feature (breakpoint count in one or both flanking regions of a predetermined length). The features may not be quantified for diploid segments. In other words, the methods of the present disclosure may comprise quantifying a plurality of copy number features for one or more non-diploid segment, such as e.g. each non-diploid segment in a copy number profile. The copy number changepoint feature may be quantified for the first segment of any chromosome by as the copy number change relative to the diploid state (i.e. 2 is subtracted from the copy number value of the segment). In other words, the changepoint feature may be quantified by subtracting 2 from the absolute copy number of the first segment of any chromosome if said segment is not a normal segment. This assumes that the (non-existing) previous segment was a normal segment (copy number 2) if the segment is not a normal segment. The copy-number changepoint feature may be quantified for a current segment relative to the upstream or downstream segment of the current segment. In particular, the copy-number changepoint may be quantified relative to the upstream (left) neighbouring segment when the changepoint value relative to the upstream segment is at or higher than a predetermined threshold (e.g. -3 or more, such as e.g. -3, -2, -1, 1, 2, 3, etc.). The copy-number changepoint may be quantified relative to the downstream (right) neighbouring segment when the changepoint value relative to the upstream segment is lower than the predetermined threshold (e.g. less than -3, such as e.g. -4, -5, etc.). When the copy number changepoint relative to the downstream neighbouring segment is also lower than -3, the copy number changepoint may be calculated by removing two from the copy number value of the current segment (i.e. calculating the difference between (current segment copy number) and (diploid state)). In any embodiment, the copy number changepoint may be quantified as an absolute value. In other words, a relative copy number changepoint between a current segment and a neighbouring segment may be calculated and the absolute value of this may then be taken as the copy number changepoint for the current segment. This approach differs from the approach in Drews et al. 2022 in which the changepoint captured the difference in absolute copy number compared to the left neighbouring segment without taking into account whether the relative change was a positive or a negative value. The approach proposed herein advantageously avoids linking an event next to a focal amplification to a replication stress signature, as was the case in the previous approach since segments with low copy number value next to a focal amplification were defined by having high copy number change. In other words, the left segment may be used by default unless it is determined that the left segment is likely a segment with focal amplification. In this case, the right segment may be used unless it is determined that the right segment is also likely a segment with focal amplification. In such cases the copy number changepoint may be calculated relative to a diploid state.

At step 18, a metric indicative of association between one or more signatures of chromosomal instability and each segment is determined using the quantified features for the segment. A signature of chromosomal instability comprises a set of weights for each of a plurality of components each associated with a copy number feature of the set of copy number features, wherein the weights are indicative of the probability that a mutational process associated with the signature generates a segment having characteristics defined by the component. Thus, step 18 may comprise obtaining, for each segment, a value for each of the plurality of components for the segment. Each component defines characteristics of a segment as a predetermined distribution of values of a copy number feature. Determining a metric indicative of association between a signature of chromosomal instability and a segment may comprise determining the probability of the copy number features of the segment belonging to each of a set of predetermined distribution associated with respective components of the plurality of components. Thus, the probability of the feature value from a segment belonging to the predetermined distribution associated with the component may be determined. This may be performed for each component, thereby obtaining a an "Event-by-component" probabilities vector for each segment, or an event-by-component probability matrix comprising respective vectors for each of a plurality of segments from the same copy number profile. These probabilities may be referred to as "posterior probabilities". Thus, step 18 may comprise quantifying the posterior probabilities of each feature value belonging to each of a set of predetermined distributions, also referred to as "components". The components may have been identified empirically by quantifying the copy number features in a plurality of tumour samples. Each copy number feature may be associated with a plurality of components. A posterior probability may be obtained for each component and for each event. Further, a summarised measure across the copy number profile may be obtained for each such component, such as e.g. by summing the posterior probabilities obtained for each event for the component (step 20). The use of empirically identified components means that the features are truly reflective of biological processes rather than arbitrary categories that while convenient to manipulate, may not be biologically relevant. For example, a set of predetermined distributions may be identified by applying mixture modelling on a data set comprises the quantified set of copy number features for a plurality of tumour samples. This enables the identification of the true states of the copy number features present in tumours, and hence the quantification of the evidence for the presence of these states in a new sample to be analysed. The use of posterior probabilities of each feature value belonging to each of a set of predetermined distributions advantageously enables the method to deal with uncertainty in the assignment of a segment to a state (i.e. uncertainty in whether the segment provides evidence for the presence of a particular category of CIN events characterised by one of the distributions for a copy number feature). This means that the method is by design able to deal with inevitable noise in the data, resulting in a more accurate characterisation of the sample. The set of predetermined distributions for a copy number feature may be a set of Gaussian distributions for any feature that is quasi-continuous, such as e.g. the segment size or changepoint feature. The set of predetermined distributions may be a set of Poisson distributions for any count feature, such as e.g. the breakpoint per side feature. A set of predetermined distributions may have been obtained using a mixture modelling technique. A quasi-continuous feature may be any feature that is not a count feature, such as e.g. segment size and copy number changepoint. For example, the segment size feature may be quantified for an event by obtaining posterior probabilities (or for a copy number profile as sum-of-posterior probabilities summed across copy number events in the copy number profile) for each of a plurality of Gaussian distributions, such as e.g. between 20 and 25 Gaussian distributions (e.g. 22 Gaussian distributions). As another example, the copy number changepoint feature may be quantified by obtaining posterior probabilities (or for a copy number profile as sum-of-posterior probabilities summed across copy number events in the copy number profile) for each of a plurality of Gaussian distributions, such as e.g. between 5 and 15 Gaussian distributions (e.g. 10 Gaussian distributions). As another example, the breakpoint per side feature may be quantified by obtaining posterior probabilities (or for a copy number profile as sum-of-posterior probabilities summed across copy number events in the copy number profile) for each of a plurality of Poisson distributions, such as e.g. between 5 and 15 Poisson distributions (e.g. 9 Poisson distributions). The precise number of distributions used may depend at least in part on the number and diversity of samples used to obtain the signatures. In the examples provided herein, the inventors used a very large number of samples from a wide variety of tumour types, enabling them to provide a nuanced picture of the behaviours of copy number features in cancer, resulting in a higher number of components identified than e.g. in Macintyre et al., 2018. The parameters of each of the predetermined distributions (such as e.g. mean and variance for a Gaussian distribution, $\lambda$ for a Poisson distribution) may have been determined as part of the process of obtaining the signatures of chromosomal instability. The parameters of each of the predetermined distributions (such as e.g. mean and variance for a Gaussian distribution, $\lambda$ for a Poisson distribution) may have been determined by fitting mixture models to the quantified set of copy number features in the plurality of tumour samples from which the signatures of chromosomal instability have been obtained. In the examples below, these parameters were obtained by analysis of over 6000 samples from the Cancer Genome Atlas (TCGA). As the skilled person understands, slightly different parameters may be obtained when using different data. The parameters of each of a set of Gaussian distributions may have been obtained using a Variational Bayes Gaussian mixture model. In particular, the parameters of Gaussian distributions (for example for features such as segment size and changepoint) may have been obtained by fitting Dirichlet-Process Gaussian mixture models using variational inference. The parameters of each of a set of Poisson distributions (for examples the breakpoint per side feature) may have been obtained using a Poisson mixture model and/or using predetermined parameters. The parameters of the components used in the examples described herein are provided in **Table 1.** Thus, in embodiments, step 16 uses the components defined in **Table 1** or corresponding components defined using a different cohort of samples. Corresponding components refer to components defined by distributions that correspond to the distributions of the components in **Table 1,** but where the exact parameters of each distribution may vary. The component parameters for the changepoint and segment size features in **Table 1** are as in Drews et al. 2022 and WO 2023/057392. The breakpoints per side feature is new to the present disclosure. In embodiments, the set of signatures used at steps 18 and 20 include one or more of the signatures in Drews et al. 2022 (e.g. signatures selected from CX1 to CX17, optionally excluding CX6). Aetiologies associated with these signatures are provided in **Table 2.** The full definitions of these signatures are provided in Drews et al. 2022 and WO 2023/057392 (see Table 7 of WO 2023/057392). In embodiments, signatures corresponding to those in WO 2023/057392 (see Table 7 of WO 2023/057392) in the new feature space described herein are obtained by applying linear combination decomposition using a cohort of samples with known exposures to the signatures. In other words, signature definitions can be identified as those that solve the minimisation problem min($\|E*SbC-PbC\|$) with constraints on nonnegativity of *SbC,* with E and *PbC* being known. For example, linear combination decomposition as implemented in R package YAPSA (Hübschmann, D. et al. 2021) may be used for this purpose. Corresponding signature definitions according to the present disclosure are provided in **Table 3.** As mentioned above, the process of defining the signatures in Drews et al. 2022 in the new feature space may be performed using one or more or all of CX1 to CX17, excluding CX6.

Step 18 (determining a metric indicative of association between a signature of chromosomal instability and a segment using the quantified features for the segment) may comprise multiplying probabilities associated with each of the plurality of components for the segment by the corresponding weight in the signature, and summing up the resulting values. As explained above, the probability associated with a component for the segment are typically the probability of a segment with the quantified feature having been drawn from the distribution associated with the component. The resulting summed up values may be referred to as an "exposure" of the event to the respective signatures. Step 18 may further comprise normalising the signature exposures for a segment by dividing each exposure by the sum of exposures across the plurality of signatures for the segment. Alternatively, step 18 (determining a metric indicative of association between a signature of chromosomal instability and a segment using the quantified features for the segment) may comprise obtaining probabilities associated with each of the plurality of components for the segment and determining a distance between said probabilities and the weights of the signature, optionally wherein the distance is a cosine distance or a Euclidian distance.

At step 20, a sample level exposure to one or more of the signatures may be determined. This may comprise obtaining, for each component, the sum of probabilities associated with the respective component for each of a plurality of segments in the copy number profile; and

determining a linear combination of one or more signatures comprising the signature that satisfies the equation

$$PbC \approx E \times SbC \qquad \text{(Equation 1)}$$

where E is a vector of size n comprising coefficients $E_{1,...,n}$ where $E_i$ is the exposure to signature i; PbC is a vector of size c, each element in the vector representing a sum of probabilities associated with a respective component; and SbC is a matrix of size c by n, each value representing the weight of a component in a signature i. The resulting exposures may further be normalised by sample such that they sum to 1 (i.e. dividing the exposure for each signature in a sample by the sum of exposures for all signatures in the sample).

At step 22, the results of steps 18 and optionally 20 are used to determine the presence / absence of signatures (and correspondingly, the presence / absence of CIN causing processes associated with the signatures). This may comprise the step of assigning a signature of chromosomal instability to a segment of the one or more segments as the signature of the one or more signatures of chromosomal instability with the highest value of the metric for the segment. For example, the metric for a signature may be the exposure of the segment to the signature and a segment may be associated with the signature with the highest exposure for the segment. Alternatively, the metric for a signature may be a distance between the vector of probabilities associated with the plurality of components for the segment and the corresponding weights in the signature, and a segment may be associated with the signature with the lowest value of the metric for the segment (when the distance is a true distance metric such as Euclidian distance) or the highest value of the of the metric for the segment (when the distance is a similarity metric such as cosine similarity). Alternatively when the metric for a signature is a probability that the segment is associated with the signature (exposure), an event may be associated with a plurality of signatures, each with respective probabilities. As explained above, the one or more signatures of chromosomal instability may be selected from those defined in Table 3 or corresponding signatures obtained by: (i) quantifying the set of copy number features in a plurality of tumour samples, and i(ii) dentifying one or more signatures likely to result in the copy number profiles of the plurality of tumour samples by nonnegative matrix factorisation. Examples of signature and their associations are provided in Table 2. In embodiments, a subset of the signatures in **Table 3** are used. For example, a subset of the signatures that is found across a plurality of technologies (i.e. a plurality of methods for obtaining sequence data suitable for the generation of a copy number profile) may be used. These may be referred to as "universal signatures". For example, CX1-5, CX8-10, and CX13 were found by the present inventors to show robust signals across technologies (including at least array data such as SNP6 arrays and shallow whole genome sequencing data, sWGS), and were classified as universal signatures. Other signatures in Table 3 were classified as SNP6-specific signatures (CX7, CX11-12, and CX14-17). CX14 was not considered universal because its signal was captured by CX1 in sWGS data. Thus, determining the presence or absence of a signature may be equivalent to determining whether a corresponding CIN process is or has been active in the sample, or in other words determining whether the copy number alterations in the copy number profile have been generated at least in part by the CIN process. Thus, the one or more signatures comprise one or more signatures selected from: one or more signatures associated with chromosome missegregation, optionally chromosome missegregation via defective mitosis and/or telomere dysfunction, one or more signatures associated with impaired homologous recombination, one or more signatures associated with tolerance of whole genome duplication, one or more signatures associated with impaired non-homologous end joining, one or more signatures associated with replication stress, and one or more signature associated with impaired DNA damage sensing. Further, the presence / absence of a signature or corresponding process may also be determined at the sample level, by comparing the exposures obtained at step 20 to a respective predetermined threshold. The predetermined thresholds may have been obtained by performing simulations as the threshold such that a predetermined proportion (e.g. 95%) of simulated samples in which a signature is truly not present (but where the signature exposure may nonetheless be non-zero due to the presence of noise in the simulated data) are below the threshold. Examples thresholds for the signatures in Table 3 are provided in Table 4. Other thresholds may be used depending on the desired stringency in identifying the presence of a signature.

At optional step 24, the results of one or more of the preceding steps (e.g. one or more of the tumour copy number profile, quantified features for segments, quantified metrics for segments, parameters of the components used, sample exposure, identification of signatures / processes associated with an event or present in a sample etc., as well as any information derived therefrom such as a treatment recommendation or prognosis as explained by reference to Figure 2) are provided to a user, for example through a user interface, or to a computing system memory or database.

Table 1. Definition of the 43 mixture components used. Feature=One of the five fundamental features of copy number aberrations; Mean = If SD is present, then this is the mean of the Gaussian component, otherwise mean of the Poisson component; SD = If present, standard deviation of the Gaussian component; Number = Number of component used as identifiers.

| Feature | Mean | SD | Number |
|---|---|---|---|
| segsize | 38420.7697 | 15438.8759 | 1 |
| segsize | 86319.107 | 30243.0733 | 2 |
| segsize | 171234.256 | 55096.0468 | 3 |
| segsize | 427454 | 174237 | 4 |
| segsize | 918939.408 | 260126.652 | 5 |
| segsize | 1494667.9 | 404499.004 | 6 |
| segsize | 2496039.63 | 608098.846 | 7 |
| segsize | 3898217.97 | 583841 .597 | 8 |
| segsize | 4908317.82 | 398896.482 | 9 |
| segsize | 6371179 | 985163 | 10 |
| segsize | 8780945 | 1086234 | 11 |
| segsize | 13435586 | 1439442 | 12 |
| segsize | 18665397 | 1664701 | 13 |
| segsize | 22913723.2 | 1028090.2 | 14 |
| segsize | 30753807 | 2370747.45 | 15 |
| segsize | 36812267.4 | 1799660.32 | 16 |
| segsize | 44866788 | 4250506.18 | 17 |
| segsize | 57831002.6 | 3574456.9 | 18 |
| segsize | 68086495.8 | 6023604.95 | 19 |
| segsize | 81015695.9 | 3149494.22 | 20 |
| segsize | 93268268 | 10492824.6 | 21 |
| segsize | 154120037 | 27901372.7 | 22 |
| changepoint | 0.10594582 | 0.06312023 | 1 |
| changepoint | 0.34283504 | 0.1572276 | 2 |
| changepoint | 1.00818588 | 0.2528096 | 3 |
| changepoint | 1.40754134 | 0.3988254 | 4 |
| changepoint | 2.20266882 | 0.4656683 | 5 |
| changepoint | 3.20155742 | 0.32865165 | 6 |
| changepoint | 4.11442601 | 0.49429328 | 7 |
| changepoint | 5.13850887 | 1.16026304 | 8 |
| changepoint | 6.93357762 | 1.76527488 | 9 |
| changepoint | 9.96341765 | 2.61954362 | 10 |
| bpwindow | 0 | NA | 1 |
| bpwindow | 1 | NA | 2 |
| bpwindow | 2 | NA | 3 |
| bpwindow | 3 | NA | 4 |
| bpwindow | 4 | NA | 5 |

(continued)

| Feature | Mean | SD | Number |
|---------|------|-----|--------|
| bpwindow | 5 | NA | 6 |
| bpwindow | 6 | NA | 7 |
| bpwindow | 8.912199 | NA | 8 |
| bpwindow | 26.982550 | NA | 9 |

Table 2. Aetiologies of CIN signatures in Drews et al. 2022. Sign=signature. Note CX6 is not used in the present work.

| Sign | Putative cause | Pattern of Change |
|------|----------------|-------------------|
| CX1 | Chromosome missegregation via defective mitosis and/or telomere dysfunction | Whole arm/chromosome changes |
| CX2 | Impaired homologous recombination (HR) | Clustered, short to medium sized segments |
| CX3 | Impaired HR with replication stress and impaired damage sensing | Long segments with one copy change |
| CX4 | PI3K/AKT-mediated toleration of whole-genome duplication | Short and long segments with two copies changed |
| CX5 | Impaired HR with replication stress | Clustered, medium to medium-long sized segments in oscillating chains |
| CX6 | Chromosome missegregation via defective mitosis | Whole arm/chromosome changes |
| CX7 | Unknown | Segments with small changepoints |
| CX8 | Replication stress | Low level amplifications |
| CX9 | Replication stress | Mid-level focal amplifications |
| CX10 | Impaired NHEJ with replication stress | Clustered short to medium sized segments |
| CX11 | Replication stress | Clustered short segments |
| CX12 | Unknown | Short segments in long oscillating chains |
| CX13 | Replication stress | High level clustered amplifications |
| CX14 | Chromosome missegregation via defective mitosis | Whole arm/chromosome changes |
| CX15 | Unknown | Clustered medium sized segments |
| CX16 | Unknown (Impaired DNA repair?) | Clustered short segments of two to three copies |
| CX17 | Unknown (Replication stress?) | Clustered medium to medium-long sized segments of one to two copies |

Table 3. Signature by component definition matrix. CIN signatures are defined in the new feature space, which is composed by 41 components from 3 copy number features.

|  | CX1 | CX2 | CX3 | CX4 | CX5 | CX7 |
|--|-----|-----|-----|-----|-----|-----|
| segsize1 | 0 | 0 | 0 | 0 | 0.003008 | 0 |
| segsize2 | 0 | 0 | 0 | 0.000258 | 0.006984 | 0 |
| segsize3 | 0 | 0.01485 | 0 | 0.009062 | 0.012509 | 0 |
| segsize4 | 0 | 0.046256 | 0 | 0.024486 | 0.038855 | 0 |
| segsize5 | 0 | 0.046792 | 0 | 0.024972 | 0.049311 | 0 |
| segsize6 | 0 | 0.042981 | 0 | 0.024842 | 0.054903 | 0 |
| segsize7 | 0 | 0.03803 | 0 | 0.024146 | 0.056993 | 0 |
| segsize8 | 0 | 0.017155 | 0.011221 | 0.010445 | 0.036519 | 0 |

(continued)

|  | CX1 | CX2 | CX3 | CX4 | CX5 | CX7 |
|---|---|---|---|---|---|---|
| segsize9 | 0 | 0.006448 | 0.01542 | 0.002691 | 0.022418 | 0 |
| segsize10 | 0 | 0.006183 | 0.035946 | 0.003389 | 0.032475 | 0 |
| segsize11 | 0 | 0.002782 | 0.053594 | 0.001443 | 0.032137 | 0 |
| segsize12 | 0 | 0.000859 | 0.064384 | 0.002082 | 0.024896 | 0.002473 |
| segsize13 | 0 | 0.001325 | 0.048199 | 0.003212 | 0.013212 | 0.013641 |
| segsize14 | 0 | 0.002722 | 0.031708 | 0.005693 | 0.005202 | 0.014667 |
| segsize15 | 0 | 0.005238 | 0.049833 | 0.016119 | 0.002042 | 0.027837 |
| segsize16 | 0 | 0.001718 | 0.021927 | 0.013338 | 0 | 0.011535 |
| segsize17 | 0 | 0.001562 | 0.033293 | 0.015699 | 0 | 0.010037 |
| segsize18 | 0.038826 | 0 | 0.015552 | 0.009292 | 0 | 0.009259 |
| segsize19 | 0.010164 | 0 | 0.012451 | 0.009824 | 0 | 0.006003 |
| segsize20 | 0.038043 | 0 | 0.002676 | 0.009989 | 0 | 0.004069 |
| segsize21 | 0.171353 | 0 | 0.007379 | 0.013914 | 0 | 0.003872 |
| segsize22 | 0.085571 | 0 | 0 | 0.01954 | 0 | 0 |
| changepoint1 | 0 | 0 | 0.00826 | 0 | 0.007422 | 0.234523 |
| changepoint2 | 0 | 0.010465 | 0.031661 | 0.001916 | 0.035946 | 0.483507 |
| changepoint3 | 0 | 0.29153 | 0.101567 | 0 | 0.061602 | 0 |
| changepoint4 | 0 | 0.151751 | 0.05381 | 0.093501 | 0.057964 | 0 |
| changepoint5 | 0 | 0 | 0.004772 | 0.302875 | 0.049126 | 0 |
| changepoint6 | 0 | 0 | 0 | 0.021103 | 0.014631 | 0 |
| changepoint7 | 0 | 0 | 0 | 0.008041 | 0.00558 | 0 |
| changepoint8 | 0 | 0 | 0 | 0.008761 | 0.007097 | 0 |
| changepoint9 | 0 | 0 | 0 | 0.002521 | 0.005226 | 0 |
| changepoint10 | 0 | 0 | 0 | 0 | 0.003308 | 0 |
| bpwindow1 | 0.656044 | 0 | 0 | 0.011781 | 0 | 0 |
| bpwindow2 | 0 | 0.050484 | 0.17277 | 0.062918 | 0.037881 | 0.092666 |
| bpwindow3 | 0 | 0.064471 | 0.139591 | 0.066034 | 0.067347 | 0.060719 |
| bpwindow4 | 0 | 0.066314 | 0.069129 | 0.061066 | 0.073976 | 0.025022 |
| bpwindow5 | 0 | 0.059424 | 0.014858 | 0.052428 | 0.06836 | 0.00017 |
| bpwindow6 | 0 | 0.044109 | 0 | 0.039271 | 0.056362 | 0 |
| bpwindow7 | 0 | 0.026548 | 0 | 0.023343 | 0.042234 | 0 |
| bpwindow8 | 0 | 0 | 0 | 0 | 0.014473 | 0 |
| bpwindow9 | 0 | 0 | 0 | 0 | 0 | 0 |
|  | CX8 | CX9 | CX10 | CX11 | CX12 | CX13 |
| segsize1 | 0 | 0.014569 | 0.016587 | 0.121108 | 0.433208 | 0.01637 |
| segsize2 | 0 | 0 | 0.033106 | 0.144596 | 0.237703 | 0.011493 |
| segsize3 | 0 | 0 | 0.081343 | 0.187797 | 0.090704 | 0 |
| segsize4 | 0 | 0.028215 | 0.159674 | 0.136625 | 0 | 0 |
| segsize5 | 0 | 0.012417 | 0.063749 | 0 | 0 | 0 |

(continued)

|  | CX8 | CX9 | CX10 | CX11 | CX12 | CX13 |
|---|---|---|---|---|---|---|
| segsize6 | 0 | 0 | 0.006834 | 0 | 0 | 0 |
| segsize7 | 0.001034 | 0 | 0 | 0 | 0 | 0 |
| segsize8 | 0 | 0 | 0 | 0 | 0 | 0 |
| segsize9 | 0 | 0 | 0 | 0 | 0 | 0 |
| segsize10 | 0 | 0 | 0 | 0 | 0 | 0 |
| segsize11 | 0 | 0 | 0.003924 | 0 | 0 | 0 |
| segsize12 | 0 | 0 | 0.007124 | 0 | 0 | 0 |
| segsize13 | 0.002818 | 0 | 0.006677 | 0 | 0 | 0 |
| segsize14 | 0.013274 | 0.003318 | 0.002885 | 0 | 0 | 0 |
| segsize15 | 0.030114 | 0.032963 | 0.004451 | 0 | 0 | 0 |
| segsize16 | 0.020203 | 0.026273 | 0 | 0 | 0 | 0 |
| segsize17 | 0.031902 | 0.050721 | 0 | 0 | 0 | 0 |
| segsize18 | 0.01648 | 0.031824 | 0 | 0 | 0 | 0.002 |
| segsize19 | 0.012977 | 0.025013 | 0 | 0 | 0 | 0.000711 |
| segsize20 | 0.009079 | 0.009843 | 0 | 0 | 0 | 0.001096 |
| segsize21 | 0.019814 | 0.027608 | 0 | 0 | 0 | 0.00505 |
| segsize22 | 0.012258 | 0 | 0 | 0 | 0 | 0.008223 |
| changepoint1 | 0 | 0.023851 | 0.047226 | 0.027861 | 0.018339 | 0 |
| changepoint2 | 0 | 0 | 0 | 0.030561 | 0.020913 | 0 |
| changepoint3 | 0 | 0 | 0 | 0 | 0 | 0 |
| changepoint4 | 0 | 0 | 0.051714 | 0 | 0 | 0 |
| changepoint5 | 0 | 0 | 0.148069 | 0 | 0 | 0 |
| changepoint6 | 0.265062 | 0.07978 | 0.02575 | 0 | 0 | 0 |
| changepoint7 | 0.19741 | 0.094449 | 0.006663 | 0 | 0 | 0 |
| changepoint8 | 0.140058 | 0.102551 | 0.005794 | 0 | 0 | 0.024379 |
| changepoint9 | 0.083134 | 0.106124 | 0.001865 | 0 | 0.002302 | 0.075587 |
| changepoint10 | 0.02314 | 0.154128 | 0 | 0 | 0 | 0.534353 |
| bpwindow1 | 0 | 0 | 0 | 0 | 0 | 0.004852 |
| bpwindow2 | 0.063109 | 0 | 0.025255 | 0 | 0 | 0 |
| bpwindow3 | 0.035308 | 0 | 0.047763 | 0 | 0 | 0 |
| bpwindow4 | 0.015639 | 0 | 0.056399 | 0 | 0 | 0 |
| bpwindow5 | 0.006069 | 0 | 0.057753 | 0.018499 | 0 | 0 |
| bpwindow6 | 0.001117 | 0 | 0.053679 | 0.048864 | 0 | 0 |
| bpwindow7 | 0 | 0.046096 | 0.048646 | 0.062794 | 0.039005 | 0.051042 |
| bpwindow8 | 0 | 0.130257 | 0.03707 | 0.119872 | 0.086918 | 0.245133 |
| bpwindow9 | 0 | 0 | 0 | 0.101422 | 0.070908 | 0.019713 |
|  | CX14 | CX15 | CX16 | CX17 | | |
| segsize1 | 0 | 0 | 0.138313 | 0 | | |
| segsize2 | 0 | 0 | 0.03019 | 0.001848 | | |

(continued)

|  | CX14 | CX15 | CX16 | CX17 |
|---|---|---|---|---|
| segsize3 | 0 | 0 | 0.00909 | 0.00267 |
| segsize4 | 0 | 0 | 0.003088 | 0 |
| segsize5 | 0 | 0 | 0 | 0 |
| segsize6 | 0 | 0.020742 | 0.004325 | 0 |
| segsize7 | 0 | 0.072284 | 0.014543 | 0.009889 |
| segsize8 | 0 | 0.053042 | 0.018351 | 0.019246 |
| segsize9 | 0 | 0.029315 | 0.014534 | 0.010703 |
| segsize10 | 0 | 0.045143 | 0.024428 | 0.014733 |
| segsize11 | 0 | 0.040969 | 0.024514 | 0.025776 |
| segsize12 | 0 | 0.031268 | 0.01899 | 0.037424 |
| segsize13 | 0 | 0.014623 | 0.010479 | 0.042574 |
| segsize14 | 0 | 0.008339 | 0.005354 | 0.021559 |
| segsize15 | 0 | 0.014348 | 0.003237 | 0.025722 |
| segsize16 | 0.048175 | 0.002422 | 0 | 0.020824 |
| segsize17 | 0.053322 | 0.003717 | 0 | 0.024951 |
| segsize18 | 0.035299 | 0.002088 | 0 | 0.023508 |
| segsize19 | 0.044765 | 0.001008 | 0 | 0.017887 |
| segsize20 | 0.057217 | 0 | 0 | 0.005122 |
| segsize21 | 0.058474 | 0.002571 | 0 | 0.005617 |
| segsize22 | 0.189224 | 0 | 0 | 0.006861 |
| changepoint1 | 0.257408 | 0.040662 | 0.010108 | 0.003017 |
| changepoint2 | 0.109301 | 0.146915 | 0.012587 | 0 |
| changepoint3 | 0 | 0.105117 | 0.035383 | 0 |
| changepoint4 | 0 | 0.031761 | 0.065066 | 0.100747 |
| changepoint5 | 0 | 0 | 0.139367 | 0.142132 |
| changepoint6 | 0 | 0 | 0.056408 | 0.038538 |
| changepoint7 | 0 | 0 | 0.005988 | 0.026698 |
| changepoint8 | 0 | 0 | 0.008003 | 0.018706 |
| changepoint9 | 0 | 0 | 0.003473 | 0.008701 |
| changepoint10 | 0 | 0 | 0.000413 | 0 |
| bpwindow1 | 0.118391 | 0 | 0 | 0.005229 |
| bpwindow2 | 0.028425 | 0.113297 | 0.055184 | 0.121143 |
| bpwindow3 | 0 | 0.107704 | 0.080587 | 0.110349 |
| bpwindow4 | 0 | 0.071032 | 0.078136 | 0.070994 |
| bpwindow5 | 0 | 0.037088 | 0.062921 | 0.0342 |
| bpwindow6 | 0 | 0.004547 | 0.043082 | 0.002632 |
| bpwindow7 | 0 | 0 | 0.023859 | 0 |
| bpwindow8 | 0 | 0 | 0 | 0 |
| bpwindow9 | 0 | 0 | 0 | 0 |

Applications

The above methods find applications in the context of identifying signatures of chromosomal instability associated with a sample as well as individual events within a sample, such as e.g. for the purpose of characterising historical or ongoing chromosomal instability as well as tumour heterogeneity in terms of CIN.

In general, the characterisation of a DNA sample from a tumour in terms of signatures of chromosomal instability active in the sample can be used to identify a treatment for a subject with cancer, and to identify a drug target for the treatment of cancer. All of these applications are still possible using the methods described herein. Thus, the invention also provides a method of treating cancer in a subject, wherein the method comprises administering or recommending a subject for administration of a particular therapy, depending on the signature(s) of chromosomal instability that have been found to be active in the sample.

Figure 2 illustrates a method of providing a prognosis, identifying a drug target, identifying a therapy or treating a subject according to an embodiment of the present disclosure. The method may comprise optional step 30 of obtaining a DNA sample from a tumour of a subject, or from a plurality of tumour samples. Optionally, a matched normal sample may also be obtained from the subject. This may be used to provide a tumour copy number profile, as known in the art (see e.g Van Loo et al., 2010). The step of obtaining a sample from a subject may comprise physically obtaining the sample from the subject. Alternatively, the sample may have been previously obtained and no interaction with the subject may be required. In other words, obtaining a DNA sample may comprise receiving a previously acquired DNA sample. Optionally, sequence data may be obtained from the tumour (and optionally the matched normal) DNA sample(s). The step of obtaining sequence data from a DNA sample may comprise sequencing the DNA sample or analysing the sample using a genomic array. The step of obtaining sequence data from a DNA sample may comprise performing single cell or bulk DNA sequencing. Alternatively, sequence data may have been previously obtained. The sequence data may comprise bulk DNA sequencing data, single cell DNA sequencing data, or pseudo-bulk sequencing data. Pseudo-bulk sequencing data refers to sequencing data that has been obtained by combining single cell DNA sequencing data for a plurality of cells in a sample. Thus, obtaining sequence data may comprise receiving the data from one or more databases, or from a user through a user interface. The sequence data may comprise sequence data for each of a plurality of single cells. At step 32, the sample(s) is/are characterised using methods described herein such as e.g. by reference to Figure 1A. This may be performed for the sample as a whole using bulk sequencing data or pseudo-bulk sequencing data. Instead or in addition to this, this may be performed individually for each of a plurality of single cells in the sample, using respective single cell sequencing data. Thus, as a result of step 32, one or more copy number events may each be associated with one or more signatures and/or the sample as a whole or each of one or more cells of the sample may be associated with one or more signatures. In other words, exposure to one or more signatures may be determined for individual events and/or samples (where a sample in this context can refer to a whole sample or an individual cell within a sample). This can in turn be used to determine which signature(s) are associated with individual events and/or present in a sample.

Based on this determination, a subject may be classified at step 40 as likely to acquire a particular event that has been previously shown to be associated with a particular signature that is determined to be present in the sample. For example, a particular oncogene amplification event can be associated with one or more signatures, and a subject may be classified as likely to acquire the particular oncogene amplification event when the exposure of the sample to the one or more signatures is above a predetermined threshold. The predetermined threshold may comprise a plurality of respective predetermined thresholds for each of a plurality of signatures, or a single threshold that applies to a metric that is obtained as a weighted combination of the exposures of a plurality of signatures (e.g. a single predetermined threshold may be applied to a linear combination of the exposure of the sample to a plurality of signatures, where each exposure may be weighted by a corresponding predetermined weight). A sample that has exposure to the one or more signatures above the predetermined threshold may be determined as likely to acquire the oncogene amplification. May oncogene amplification events have been linked to treatment resistance and/or prognosis. Thus, this may in turn be used to provide a treatment recommendation and/or prognosis (steps 34-38 and/or 35).

Based on the determination at step 32 and/or at step 40, a subject may be classified at step 34 as being likely to respond or unlikely to respond to a particular course of treatment, where responder/non-responder status is known to be associated with exposure to one or more signatures. The particular course of treatment may target a particular gene. A signature of chromosomal instability may be known to be associated with response to inhibition of a particular gene if the exposure to the signature is significantly correlated with the effect (e.g. cellular proliferation, growth inhibition, toxicity, etc) of perturbation of the gene, for example by genetic perturbation (such as in a CRISPR essentiality screen, also referred to as CRISPR knockout screen, or in an RNAi essentiality screen) or by drug perturbation of the gene (such as in a drug response screen). At optional step 36, a particular course of treatment (which may comprise one or more different individual therapies) may be identified based on the results of step 34. For example, a subject that has been identified at step 34 as unlikely to respond to the particular course of therapy may be identified as likely to benefit from a therapy that is different from the particular course of therapy. Alternatively, a subject that has been identified at step 34 as likely to respond to the particular course of therapy may be identified as likely to benefit from a therapy that includes the particular course of therapy. At optional step 38, the subject may be treated with the therapy identified at step 36.

Instead or in addition to this, the methods described herein may be used to provide a prognosis for a subject. Thus, based on the determination made at steps 32 and/or 40, a subject may be classified at step 35 as having a good or a poor prognosis, where prognosis is known to be associated with exposure to one or more signatures. For example, a signature may be known to be associated with prognosis if samples with a high or low exposure to the one or more signatures are associated with different prognosis in a cohort of patients. In other words, a signature may be known to be associated with prognosis if samples with a good prognosis in a cohort of patients have a significantly different expected exposure to the signature than samples with a poor prognosis. The prognosis may be specific to a particular tumour type, such as e.g. ovarian cancer.

Instead or in addition to this, the methods described herein may be used to identify a drug target for the treatment of cancer. This may comprise obtaining tumour copy number profiles for a plurality of samples and characterising them at step 32 using the methods described herein. The plurality of DNA samples in this case comprise samples obtained from a tumour or a tumour cell line in which the drug target has been the subject of inhibition and for which response to inhibition of the drug target has been quantified. This may further comprise at step 37 determining whether one or more signatures of chromosomal instability are associated with response to inhibition of the drug target, wherein the presence of a signature of chromosomal instability associated with response to inhibition of the drug target is indicative of the drug target being usable for the treatment of a cancer in which the signature is active. This may further comprise optional step 39 of identifying a drug that targets the drug target, for example through a drug database or using any drug design method known in the art. Instead or in addition to this, the methods described herein may be used to identify a genomic feature that is associated with a particular signature. This may comprise obtaining tumour copy number profiles for a plurality of samples and characterising them at step 32 using the methods described herein, these embodiments step 37 may comprise determining whether one or more signatures of chromosomal instability are associated with one or more colocalised genomic features, such as e.g. epigenetic state or chromatin structure. For example, the presence of copy number events associated with a particular signature may be correlated with the presence of a particular genomic feature in a region that encompasses the copy number event or is defined by a distance relative to the copy number event. A genomic feature may be for example an epigenetic state or a chromatin state. For example, the methods described herein may be used to identify a link between a chromatin state (e.g. heterochromatin or euchromatin, presence of a particular type of DNA and.or histone modification or combination of modifications) and the activity of a particular mutational process associated with a CIN signature as described herein. This may be used for example in the context of drug screening, to characterise drugs that affect epigenetic state in terms of likely associated copy number instability processes, or in the context of characterising a patient with cancer or a cohort of patients (such as e.g. associating genomic characteristics of patients with drug responses).

Instead or in addition to this, the results of step 32 may be used at step 41 to determine which signatures are currently active in a sample and which signatures are historically active in a sample. In particular, when the determination is made for a plurality of single cells, signatures that are determined to be associated with events that are shared between a plurality of single cells in the sample (such as e.g. events that are present in a proportion of single cells above a predetermined threshold, such as e.g. more than 1 cells) may be determined to be signatures that have been active in the sample prior to a clonal expansion event. By contrast, signatures that are determined to be associated with events that are not shared between a plurality of single cells in the sample (such as e.g. events that are present in a proportion of single cells below a predetermined threshold, such as e.g. in a single cell) may be determined to be signatures that are currently active in the sample. This may in turn be used to determine a treatment response and/or prognosis for the subject, for example based on known prognosis or treatment response associated with mutational processes currently active in a sample.

Any treatment described herein may be used alone or in combination with another treatment. For example, any treatment with a drug may be used in combination with one or more chemotherapies, one or more course of radiation therapy, and/or one or more surgical interventions. In particular, any treatment described herein may be used in combination with a treatment for which the subject has been identified as likely to be responsive.

<u>Systems</u>

**Figure 3** shows an embodiment of a system for implementing any method according to the present disclosure. The system comprises a computing device 1, which comprises a processor 101 and computer readable memory 102. In the embodiment shown, the computing device 1 also comprises a user interface 103, which is illustrated as a screen but may include any other means of conveying information to a user such as e.g. through audible or visual signals. The computing device 1 is communicably connected, such as e.g. through a network 6, to sequence data acquisition means 3, such as a sequencing machine, and/or to one or more databases 2 storing sequence data. The one or more databases may additionally store other types of information that may be used by the computing device 1, such as e.g. reference sequences, parameters, etc. The computing device may be a smartphone, tablet, personal computer or other computing device. The computing device is configured to implement a method as described herein. In alternative embodiments, the computing device 1 is configured to communicate with a remote computing device (not shown), which is itself configured to implement a method as described herein. In such cases, the remote computing device may also be configured to send the result of the method to the computing device. Communication between the computing device 1 and the remote computing device may be through a wired or wireless connection, and may occur over a local or public network such as e.g. over the public internet or over WiFi. The sequence data acquisition 3 means may be in wired connection with the computing device 1 and/or the database 2, or may be able to communicate through a wireless connection, such as e.g. through a network 6, as illustrated. The connection between the computing device 1 and the sequence data acquisition means 3 may be direct or indirect (such as e.g. through a remote computer). The sequence data acquisition means 3 are configured to acquire sequence data from nucleic acid samples, for example genomic DNA samples extracted from cells and/or tissue samples. The sequence data acquisition means is preferably a next generation sequencer, but can also be an array reader. The sequence data acquisition means 3 may be in direct or indirect connection with one or more databases 2, on which sequence data (raw or partially processed) may be stored.

The following is presented by way of example and is not to be construed as a limitation to the scope of the claims.

EXAMPLES

Introduction

Example 1- Mapping signatures to individual copy number events

An approach to evaluate the origin and extent of chromosomal instability at a genome-wide level has been previously described in Drews, R. M. et al. 2022. This approach was used to identify a compendium of 17 copy number signatures. As part of this method, the copy number changes observed in a tumour were embedded into a feature space consisting of 5 fundamental copy number features (the segment size, the difference in copy number between adjacent segments, the lengths of oscillating copy number segment chains, the breakpoint count per 10 Mb, and the breakpoint count per chromosome arm). These 5 fundamental features were capable of capturing well-known copy number patterns and thus were able to distinguish different CIN types at genome-wide level.

However, this feature encoding cannot be directly applied to analyse *individual* copy number changes in a genome. In the original feature encoding the 3 copy number features used to model the distribution of copy number alterations across the genome (the lengths of oscillating copy number segment chains, the breakpoint count per 10 Mb, and the breakpoint count per chromosome arm) were encoded *per genomic region* rather than per individual copy number event. Thus, they cannot be used to map CIN signatures to each individual copy number event in a tumour.

To overcome this limitation, in the present example, the inventors designed a single new feature to replace these 3 features (Figure 4a). This new feature, called "the breakpoint per side" feature, is described below. This feature, alongside the segment size and changepoint features, can be used to recapitulate the original CIN signatures and facilitates mapping of signatures to individual copy number events. In addition to this new feature, the inventors also made slight modifications to the changepoint feature to enable single event mapping.

*New "breakpoints per side" feature*

When defining this new feature, the inventors focused on an encoding that not only captured the key properties of the 3 features that it was replacing, but also facilitated recovery of the CIN signatures with high weights for these features. Furthermore, they aimed to maintain a distinction between mutational processes leading to both clustered (i.e. replication stress) and isolated (i.e. mitotic errors) copy number alterations. In order to construct an event-based encoding, one must start with a specific copy number event in the genome. For ease in explanation, we call this event the currentCN.

To encode clustered copy number changes as previously represented by the breakpoint counts per 10 Mb and chromosome arm features, the inventors devised an encoding that took a window around the currentCN and counted the number of breaks occurring either side. The side with the maximum number of breaks was considered the count of the feature. The inventors took the maximum to account for the case where the currentCN is located at the telomere or centromere. For example, for segments located within e.g. 5Mb of the telomere or centromere, the counts would be expected to be underrepresented in the flanking region proximal to the centromere/telomere. This encoding has a high count if other copy number events are clustered with the currentCN and 0 if there are no neighbouring copy number events. The inventors opted for a 5 Mb window flanking each side of the currentCN. While this doesn't completely represent the breakpoint per chromosome arm feature, they reasoned that it was a good compromise between the two original breakpoint features (the breakpoint count per 10 Mb, and the breakpoint count per chromosome arm). This feature is able to capture more information than just the breakpoint count per 10Mb feature (although it does also capture the information capture by the latter) because if the currentCN is large then the counts effectively span a much larher region than 10Mb. For example, if the currentCN is 40MB, taking +5MB flanking regions means that the total region being interrogated is 50MB (close to the size of a chr arm).

Fortunately, this encoding also managed to capture information encoded by the previous length of oscillating chains feature: neighbouring copy number events of the same size that are part of an oscillating chain will have the same number of breaks in the 5 Mb flanking region, and will therefore cause an overrepresentation of a specific count value in the new feature. Hence, the encoding of the breakpoints per size feature also captures oscillating chains.

As was also done in Drews et al. 2022, diploid segments were not considered and thus the breakpoints per size feature was only extracted for non-diploid copy number events.

To test the robustness of this new feature encoding, the inventors rederived signatures and compared their activities to those obtained by using the original encoding (see below).

*Modifications to the changepoint feature*

This feature was also included in the feature space in Drews et al. 2022, however the feature encoding has been modified in order to avoid linking an event next to a focal amplification to a replication stress signature. In the original feature encoding, the changepoint captured the difference in absolute copy number compared to the left neighbouring segment without taking into account whether the relative change was a positive or a negative value. Therefore, segments with low copy number value next to a focal amplification were defined by having high copy number change, and therefore were assigned to one of the amplification-related signatures.

For non-diploid segments, the relative changepoint value is now calculated with respect to the segments on both sides (even if the neighbouring segments are diploid). The changepoint value is assigned with respect to the left neighbouring segment when this value is equal or higher than - 3. In case this condition is not fulfilled, the changepoint value is assigned with respect to the right neighbouring segment. Then, for those segments with relative changepoint values lower than -3 (i.e. segments flanked by focal amplifications on both sides), the changepoint value is recalculated by subtracting 2 (normal copy number) from the copy number value of this segment. In other words, this subtracts the normal state from the current copy number (as if the segment was flanked by normal segments), to avoid assigning segments that are between two focal amplificaitons to a replication stress signature. Finally, relative changepoint values are transformed to absolute changepoint values. Making these changes improves the precise mapping of each event to a specific signature. This is not as important when signatures are evaluated at the genome wide level, but improves the accuracy at the single event level.

As in Drews et al. 2022, no changepoint value is extracted for diploid segments. For non-diploid segments located at the beginning of the chromosome, the changepoint value is calculated with respect to the normal copy number value (i.e. 2 is subtracted from the copy number value).

This change had overall little impact on the signature activities quantified for the TCGA samples using the original feature encoding (**Figure 5,** see Example 2 for methods of TCGA data analysis). The major difference was a merge of mitotic signatures CX1 and CX6 (the activities of these signatures are highly correlated and it is therefore possible that these two signatures are in fact the same signature artificially split during the NMF process due to limited data input). For that reason, signature CX6 is excluded from any downstream analyses.

*Absolute copy number feature*

As explained in Drews et al. 2022, the inventors made a deliberate decision to exclude the absolute copy number of a segment as a feature for decoding chromosomal instability. This choice was motivated by the need to ensure robustness in the presence of whole-genome doubling (WGD). Through an extensive examination of the ovarian copy number signatures (Macintyre *et al.* 2018), the inventors discovered that the inclusion of the copy number feature could artificially split a signature based on ploidy status, even when the underlying mutational process was the same. For instance, a single copy loss on a whole genome duplication (WGD) background would result in a copy number state of 3, while the same deletion on a non-WGD background would yield a copy number state of 1. Consequently, a segment loss could be encoded by two distinct signatures, despite originating from the same mutational process that generates deletion patterns. As in Drews et al. 2022, the new feature encoding described herein exclusively incorporates the changepoint feature, which quantifies changes in copy number relative to adjacent segments. By using simulated genomes, the inventors explicitly confirmed that the new feature encoding remains resilient to the influence of WGD effects (see section "Assessing feature encoding performance"). Furthermore, the approach to assign CIN signatures to individual copy number events was also robust to the WGD effects.

*Feature distribution and modelling*

Each feature was split into categories or components by applying mixture modelling to the feature distributions (see Figure 4a). For the segment size and changepoint feature, the same mixture components used in Drews et al. 2022 were used (see **Table 1**). For the new feature (bpwindow), the inventors estimated Poisson mixture models with the flexmix package in R (Grün, B. & Leisch, F. 2008), and used the Bayesian Information Criterion (BIC) to decide on the number of components. The algorithm was initially run using all data with the number of components ranging from 1 to 10. This only yielded 2 components which was not sufficient to represent range of values observed (**Figure 6**). Therefore, given that 96% of the data presented less than 6 breakpoints at 5 Mb window per segment side, the inventors decided to manually define 7 components representing from 0 to 6 counts (see **Table 1**). Then, they applied mixture modelling to the remaining 4% of the data and derived 2 additional components, resulting in a total of 9 components for the breakpoint counts at 5 Mb window per segment size feature (see Table 1).The Lambda/Mean values of each of these distributions are provided in Table 1. The bpwindow components with lambda values from 0 to 6 were manually predetermined. For the last two components, the inventors run flemix for getting lambda values with the following parameters: MINCOMP=1, MAXCOMP=10, FITDIST="pois", PRIOR=0.001, NITER=1000, DECISION="BIC", SEED=77777. Note that other parameters may be used to identify mixtures of Poisson distributions for events that present more than 6 breakpoints per 5Mb, as well as different cohorts of samples, which would result in Poisson distributions with slightly different lambda values.

In total, this resulted in a feature space defined by 41 mixture components (22 from the segment size feature, 10 from the changepoint feature, and 9 from the breakpoints per side feature).

*Event-by-component probabilities matrix*

Once components were derived, the inventors computed the probability for each copy number event to belong to each component per feature, resulting in a 1x41 dimensional vector of posterior probabilities. For a given sample, all probability vectors were combined in an event-by-component probabilities matrix, which served as the input matrix for mapping each individual copy number event to a specific CIN type.

*Event-by-signature scores matrix*

For each signature and copy number event, the inventors multiplied the component probabilities vector by the signature definition (signature definition in the new feature space, see Table 3 - obtained by linear combination decomposition given a sample level input matrix in the new feature space and the signature definitions in Drews et al. 2022 in the original feature space). Then, they summed up the resulting vector to calculate a signature score per event. In some cases (not used in the present examples), the resulting event-by-signature matrix was multiplied by the sample-level signature activities (see section "Sample-level signature quantification") to compute activity-corrected signature scores for each copy number event.

Signature scores per event were finally normalised per signature. This is done per event so that the signature scores sum to 1, i.e. the score for each signature for a specific event is divided by the sum of scores for all signatures for the event.

*Deriving novel signatures*

The inventors aimed at defining a compendium of CIN signatures in the new feature space (see Figure 4b). In the present examples, the inventors used the signatures in Drews et al. 2022 in the new feature encoding. However, it is also possible to use the new feature encoding to derive signatures from scratch (e.g. using NMF) as described in Drews et al. 2022. To derive signature definitions, they used the 6,335 TCGA samples with sufficient evidence of chromosomal instability (≥20 CNAs) according to the threshold estimated in Drews et al. 2022. in the context that the activities of the original copy number signatures are already known for all 6,335 samples, the inventors used the LCD function from YAPSA (Hübschmann, D. et al. 2021) for deriving signature definitions given the new feature space input matrix (6,335 patients by 41 components sum-of-posterior matrix).

However, the novel feature encoding introduces changes in the input matrix that need to be taken into account for following this procedure. As stated before (see section "Copy number feature encoding: "Modifications to the changepoint feature"), the inventors have modified the original encoding for the changepoint feature, which limits their ability to distinguish between mitotic signatures CX1 and CX6. This precludes the use of the original signature activities in Drews et al. 2022 as the gold-standard activities for deriving event-level signatures.

After excluding CX6 from the signature definition matrix, the final signature activities for all 6,335 TCGA samples with detectable chromosomal instability were computed using the linear combination decomposition (LCD) function from YAPSA on the input matrix. This input matrix included the sum-of-posterior probabilities for components of the 5 original features but applying the new changepoint encoding.

As described in Drews et al. 2022, the inventors computed and applied signature-specific thresholds to clarify small signature activities. The activity matrix was finally normalised per sample to obtain the new gold-standard activities for all TCGA samples. This gold-standard sample-by-activities matrix was used for deriving the definitions of the novel signatures, as explained above, using linear combination decomposition. Novel signatures (defined in **Table 3**) captured the same signal of different CIN types as the original compendium of copy number signatures in Drews et al. 2022 (**Figure 7**).

*Sample-level signature quantification*

After defining CIN signatures that can be mapped to individual events, the final signature activities for all 6,335 TCGA samples were computed using the linear combination decomposition function from YAPSA on the input matrix (sample-by-component sum-of-posterior matrix). This input matrix was derived by summing up the event probability vectors per sample (i.e. this is a matrix comprising, for each sample, a vector that has activities that are the sum of a row of the event-by-signature matrix (i.e. sum of all event specific activities for a single signature) prior to any adjusting based on sample-level signature and normalising by signature). Indeed, as the linear decomposition is not exact, the sample level activities are re-computed given the signatures defined in the new feature space. For new samples, the component vector (new encoding) is computed, then the new signature definitions (Table 3 or any equivalent signature definitions using the new feature encoding) are used to estimate the activity using linear value decomposition (e.g. as implemented in YAPSA).
Following the same procedure as in our Drews et al. 2022, the inventors identified signature-specific thresholds to be confident in small signature activities. Briefly, the inventors performed 1000 simulations whereby noise was added to the input components. For signatures with a true zero value, the non-zero simulated values were fittered with a single Gaussian distribution using Mclust from the mclust R package, v5.4.6. A threshold was estimated at 95% of this distribution and activities to zero if they were below the signature-specific threshold (**Table 4**). The sample-by-activity matrix was finally normalised per sample (sample-level activity matrix from here) - dividing by the sum of all signatures for that sample so the signature activity vector sums to one.

**Table 4.** Signature-specific thresholds for setting zero activity values.

| Signature | Threshold |
| --- | --- |
| CX1 | 0.003483 |
| CX2 | 0.002938 |
| No, CX3 | 0.008615 |
| CX4 | 0.004013 |
| CX5 | 0.007166 |
| CX7 | 0.001442 |
| CX8 | 0.00198 |
| CX9 | 0.002412 |
| CX10 | 0.00235 |
| CX11 | 0.00128 |
| CX12 | 0.001194 |
| CX13 | 0.0006052 |
| CX14 | 0.0009475 |
| CX15 | 0.005096 |
| CX16 | 0.004579 |
| CX17 | 0.01085 |

Example 2 - Robustness analysis of new CIN feature encoding

In this example the inventors evaluated the performance of the new signature mapping method described in Example 1 in terms of its ability to capture types of CIN samples in simulated data, correctly assign these types of CIN to individual events, and perform well across a range of technologies and extent of CIN.

**Methods**

*See Example 1.*

*Data.* All data used in the present examples are publicly accessible, except for the PCAWG raw data (WGS). The access to the TCGA portion of the PCAWG data can be obtained by a Data Access Request through the Database of Genotypes and Phenotypes (www.cancergenomicscloud.org/controlled-access-data). The TCGA copy number profiles used here were from Drews et al., 2022 (files hosted on github.com/VanLoo-lab/ascat). TCGA clinical data is available at TCGA Research Network: www.cancer.gov/tcga. PCAWG copy number profiles were from Gerstung, et al. 2020; Dentro, et al. 2021 (Files hosted on dcc.icgc.org/releases/PCAWG). Clinical annotation for PCAWG data is available from Pan-Cancer Analysis of Whole Genomes Consortium (2020). Files hosted on dcc.icgc.org/releases/PCAWG/

*Software.* If not otherwise stated, all analyses were performed using the free statistical software R (v>4.0). If functions outside the standard packages were used, they are mentioned and cited throughout the text. The following packages were used: in this Example and Example 1: YAPSA v. 1.22.0 (Hübschmann, D. et al. 2021), flexmix v. 2.3-19 (Grün, B. & Leisch, F., 2008).

*Sequence read alignment.* Reads were aligned as single-end against the human genome assembly GRCh37 using BWA-MEM (v0.7.17)(Li and Durbin, 2009). Duplicate reads were then identified and marked using samtools-markdup (v1.15)(Li et al. 2009).

*Generating copy number profiles.* After alignment, absolute copy number profiles were fitted from sequencing data generated across different high-throughput technologies (method applied for each dataset is detailed below). Following absolute copy number fitting, samples were rated using a star system as previously described (Macintyre, G. et al. 2018; Madrid, L. et al. 2023). Briefly, samples with noisy or flat copy number profiles where absolute copy number cannot be estimated are given 1-star, samples with fittable absolute copy number but with some noise are given 2-star, well fit samples without noise are given 3-star. Those with 1-star were discarded for downstream analyses. In addition, samples without the appropriate read counts (15 number of reads per bin per chromosome copy) for supporting the fitting were also discarded.

*Absolute copy number fitting from sWGS.* We used the QDNAseq R package (Scheinin, I. et al. 2014) to count reads within 30 kb bins (resolution threshold determined to accurately call copy number signatures in Drews et al. 2022). Bins mapped to centromeres and regions of undefined sequence in the reference genome hg19 were excluded. Read counts were then corrected for sequence mappability and GC content, followed by copy number segmentation. After the profile was segmented, we inferred absolute copy numbers for combinations of purity and ploidy values ($0.05 < purity < 1$, and $1.5 < ploidy < 8$) as follows:

$$cn = \frac{1}{purity} \times \left( \frac{r}{d} - 2 * (1 - purity) \right)$$

where *purity* is the fraction of tumour cells in the sample, *r* is the read counts, and *d* is a constant proportional to the read depth and the average absolute copy number of tumour cells in the sample (*ploidy*):

$$d = \frac{r}{(ploidy \times purity + 2 \times (1 - purity))}$$

The optimal mathematical solution was considered the one with the lowest root mean squared deviation (RMSD) between the non-rounded and the rounded copy numbers for all bins. All solutions were manually inspected to either confirm or correct for more appropriate fits. For pure cancer models (i.e. cell lines, single cells), a range of purities from 0.9 to 1 for fitting absolute copy numbers can be used instead.

*Absolute copy number fitting from single cell WGS.* Although no single cell data was used in the present examples, it is possible to infer CN profiles from such data and apply the emthdos described herein in the same was as described for WGS. For example, to infer copy number profiles from single cell DNA sequencing data, one can define 100 kb windows along the genome and apply the same methodology described in the "Inferring absolute copy number from sWGS" section.

*Absolute copy number fitting from pseudobulk WGS.* As mentioned above, single cell sequencing data can be used to obtain single cell and pseudobulk CN profiles. For pseudobulk profiles, it is possible to reconstruct bulk copy number profiles by concatenating reads of all single cells sequenced from a specific sample. One can then downsampl pseudobulk bams to appropriate read counts based on the bin size (30 kb), purity and ploidy. Absolute copy number fitting can then be performed as described in the "Inferring absolute copy number from sWGS" section.

**Results**

*Assessing feature encoding performance*

To evaluate the robustness of the new feature encoding described in Example 1, the inventors used a dataset of 240 simulated genomes which were generated in Drews et al. 2022. Briefly, they simulated the activity of five CIN types by introducing copy number changes into diploid genomes. These CIN types included: chromosome missegregation via mitotic errors (CHR), large-scale state transitions via homologous recombination deficiency (LST), focal amplification via ecDNA circularisation and amplification (ecDNA), early whole-genome duplication via cytokinesis failure (WGD early), and late whole-genome duplication via endoreduplication (WGD late). For any given sample, three of the five mutational processes were active. Half of the samples had one dominant signature and the other half had two. Each mutational process was designed to be dominant in a specific subset of samples (N=100 for CHR, ecDNA and LST; N=96 for WGD early; N=24 for WGD late).

The inventors applied the new feature encoding described in Example 1 to the simulated genomes to generate a sample-by-component sum-of-posteriors matrix. Then, the *NMF* package in R (with the Brunet algorithm specification) was used to deconvolute the sample-by-component sum-of-posteriors matrix into a sample-level activity matrix and a signature definition matrix. A signature search range of 3 to 6 was applied, running the NMF algorithm 1,000 times with different random seeds. The optimal number of signatures was based on achieving stability in cophenetic, dispersion, and silhouette coefficients. These evaluation metrics were computed for the definition matrix (basis), the activity matrix (coefficients), and the connectivity matrix (consensus). This consensus matrix represented sample clusters based on their predominant signature across the 1,000 runs. The optimal solution was defined as the smallest number of signatures that achieved stability in the cophenetic, dispersion, and silhouette coefficients; but also maximised sparsity without surpassing the sparsity observed in the randomly permuted matrices. This process yielded a total of 5 signatures (**Figure 8**). **Figure 8** shows a collection of metrics typically used to determine the optimal number of signatures (factorisation rank). Specifically this plot represents a comparison of signature number (x axis) across four measures for determining optimal signature number. The circle and solid lines represent the results from the samples run, whereas the triangles and dotted lines represent results from 1,000 randomly permuted matrices (these can be considered a null measure). Here basis refers to the signature-by-component matrix, coefficients refers to the patient-by-signature matrix, and consensus refers to the connectivity matrix of patients clustered by their dominant signature across 1,000 runs. The best fit is the run that showed the lowest objective score across the 1,000 runs. A value of 5 defines the point of stability in the cophenetic, dispersion and silhouette coefficients and is the maximum sparsity achievable above the null model for the basis matrix.

Next, the inventors assessed whether the identified signatures effectively captured the simulated CIN types. They observed a strong alignment between the definitions of the identified signatures and the expected definitions of the simulated CIN types within the feature space (average cosine similarity of 0.66; **Table 5**). The expected definitions were generated by adding weight to those components that reflect the mutational process (adding 1 to those components that define a simulated CIN type, and 0 to those components that do not define it, then normalising the definition vector to sum to 1). The signature activities largely agreed with the simulated distributions across samples, resulting in an overall accuracy of 70.5% (296/420) in identifying samples with a dominant signature (**Figure 9**). The inventors also compared activities of identified signatures by using the original and the new feature encoding. This analysis revealed that both feature spaces were equally proficient in extracting CIN signals, as indicated by an average Pearson's r of 0.71 (**Figure 10).** Overall, these results demonstrate that the new feature encoding described herein effectively accomplishes both identifying signatures of mutational processes and quantifying their corresponding activities.

**Table 5.** Similarity between expected and observed definitions of identified signatures.

| Signature | Cosine similarity |
|---|---|
| HRD/LST | 0.80 |
| ecDNA | 0.58 |
| CHR | 0.65 |
| Early WGD | 0.67 |
| Late WGD | 0.61 |
| **Average** | **0.66** |

*Assessing signature assignment to individual events*

The new approach described in Example 1 for mapping CIN signatures to individual copy number events outputs a score that reflects the likelihood of an event being attributed to each signature. This flexibility accommodates situations where copy number events exhibit features resembling more than one signature, and also avoids assigning a specific CIN type to a copy number event which has been produced by an uncertain or unrepresented mutational process. Note that there are multiple options to map signatures to events, including: 1) assign to the event a probability of being caused by a signature (as described in example 1), and 2) assign a specific signature to the event by selecting the signature with the highest probability. The process of computing the signature probability or score of an event is by multiplying "event by component" vector to the "signature by component" vector. These scores can be but do not need to be corrected by the sample-level activity. Another possibility to map signatures to events would be to assign to an event the signature with the most similar definition compared to the "event by component" vector (e.g. using cosine similarity, euclidean distance, etc).

In an attempt to assess the reliability of the proposed mapping approach, the inventors allocated each copy number event to the signature with the highest associated probability. Subsequently, they assessed the accuracy of these assignments by calculating the fraction of events displaying similar features to the definition of the signature assigned. The signature with the highest cosine similarity in comparing the event-by-component vector and the signature-by-component vector was considered as the most similar. This analysis revealed an overall accuracy of 72.2% (479,565 out of 647,630 events), denoting a substantial alignment between the mapped signature and features defining an individual copy number event. **Table 6** summarises mapping accuracy for each CIN signature.

Table 6. Fraction of events correctly assigned to the CIN signature with the most similar definition.

| CIN signature | Events correctly assigned (%) |
| --- | --- |
| CX1 | 52.3 |
| CX2 | 75.8 |
| CX3 | 76.8 |
| CX4 | 78.1 |
| CX5 | 100 |
| CX8 | 59.3 |
| CX9 | 60.5 |
| CX10 | 89.0 |
| CX13 | 57.9 |

*Assessing number of individual events required for sample-level signature quantification*

The inventors performed an analysis to determine the minimum number of copy number events required within a sample to accurately replicate its signature composition. To achieve this, they progressively increased the number of events of a sample profile via random selection. They then quantified sample-level signature activities by using the linear combination decomposition function from YAPSA on the input matrix (sample-by-component sum-of-posterior matrix). In each iteration of this decomposition, they expanded the information within the input matrix by progressively introducing an increasing number of events, ranging from 1 to 100. They then evaluated whether a sample's signature composition (defined by its three dominant signatures) was correctly captured depending on the number of events. This procedure was repeated 1000 times with 1000 TCGA samples being randomly selected for each run. As little as 15 copy number events were sufficient to correctly decompose >90% cases (**Figure 13**).

This procedure was also expanded to encompass individual signatures. For each specific signature, the inventors performed random selection of copy number events from the subset of 200 TCGA samples exhibiting the highest signature activity. Then, they quantified sample-level signature activities and evaluated the success rate of detecting the signature. This procedure was done 100 times per signature. The results indicated that an average of 7 copy number events was sufficient for calling an individual signature with >90% of accuracy (**Figure 14**).

*Sample-level signature stability across technologies*

CIN signatures were derived using TCGA SNP 6.0 array data because this represents the largest pan-cancer collection of high-resolution copy number profiled tumours to date. In Drews et al. 2022, the inventors evaluated the robustness of the approach across different high-throughput sequencing technologies by comparing signature definitions and activities. As expected, they saw a drop in similarity scores for the activities and signature definitions between SNP6 and sWGS data (Drews et al. 2022).

Here, the inventors extended this analysis by means of testing the ability to capture the compendium of signatures in sWGS. For this analysis, the inventors used 478 TCGA samples with detectable levels of CIN that were also part of the PCAWG project. WGS data from the 478 TCGA/PCAWG samples was downsampled to 15 number of reads per bin per chromosome copy (according to the ploidy, purity and bin size) for mimicking sWGS. The inventors fitted absolute copy number profiles (see section "Absolute copy number fittings from sWGS"), extracted the 5 original copy number features but using the new changepoint encoding (see section "Copy number feature encoding: Changepoint" in Example 1), quantified activities for the 16 compendium signatures (CX6 excluded) using YAPSA, and compared them to the SNP6-derived signature activities using Kendall's tau. This looks at the effect of the changes to the changepoint feature.

This analysis showed the deterioration of some signatures across technologies (**Figure 11**). These deteriorations were not due to methodology but rather due to differences in how the copy number is measured by these technologies. All signatures would be detectable given a sufficient number of training samples for that technology. Fortunately, this deterioration was only observed for tumour type-enriched signatures, with the remaining signatures showing robust signals across technologies. Signatures showing robust signals across technologies were classified as universal signatures (CX1-5, CX8-10, and CX13) whereas others were classified as SNP6-specific signatures (CX7, CX11-12, and CX14-17). CX14 was not considered universal because its signal was captured by CX1 in sWGS data. It is important to note here, that SNP6-specific signatures can also be detected in sWGS, it is just that the copy number changes captured by the signatures manifest differently across the two technologies (as shown In Figure 10 and in Drews et al. 2022, Supplementary Fig. 46-47).

*Sample-level signature stability across bin resolutions*

Given that the gold-standard bin size for quantifying the compendium of CIN signatures in Drews et al. 2022 is 30 kb, and the bin size used for deriving copy number profiles from WES data is 50 kb, the inventors explored the robustness of the novel signatures across bin resolutions by comparing activities.

Here, they segmented copy number profiles from the 478 TCGA/PCAWG samples using 8 different bin sizes (30, 40, 50, 60, 70, 80, 90 and 100 kb). Once absolute copy number profiles were fitted, the inventors quantified the novel signatures developed in this work per sample. They then computed cosine similarities of SNP6-derived signature activities with those obtained at each bin resolution (**Figure 12a**). In addition, they compared signature activities derived at 30 kb (gold-standard) and 50 kb (resolution used for WES data) using Kendall's tau (**Figure 12b**). Altogether, these comparisons indicate that the novel signatures are stable across bin resolutions.

Example 3 - Applications of the novel signature method

The novel signature method can be used in a variety of contexts. In particualr, the approach is beneficial in the context of analysing single cell sequencing data. Whe analysing bulk sequencing data it is only possible to look at the "average" tumour genome. However, when looking at single cells it becomes possible to analyse and compare individual events in individual cells. However, previous CIN signatrues were not able to fully make use of this information since they could not assign signatures to individual events. By assigning signatures to individual events it is possible to detect the CIN type(s) that is/are active in the tumour at the current time, and therefore identify the processes that are causing the acquisition of new copy number events. These "ongoing CIN signatures" can then be used to predict response to drugs.

References

Raine KM, et al. ascatNgs: Identifying Somatically Acquired Copy-Number Alterations from Whole-Genome Sequencing Data. Curr Protoc Bioinformatics. 2016 Dec 8;56:15.9.1-15.9.17.

Van Loo, P. et al. Allele-specific copy number analysis of tumors. Proc. Natl. Acad. Sci. U. S. A. 107, 16910-16915 (2010).

Langmead, B., Trapnell, C., Pop, M. et al. Ultrafast and memory-efficient alignment of short DNA sequences to the human genome. Genome Biol 10, R25 (2009).

Carter, S., Cibulskis, K., Helman, E. et al. Absolute quantification of somatic DNA alterations in human cancer. Nat Biotechnol 30, 413-421 (2012).

Favero F, Joshi T, Marquard AM, Birkbak NJ, Krzystanek M, Li Q, Szallasi Z, Eklund AC. Sequenza: allele-specific copy number and mutation profiles from tumor sequencing data. Ann Oncol. 2015 Jan;26(1):64-70.

Adalsteinsson, V.A., Ha, G., Freeman, S.S. et al. Scalable whole-exome sequencing of cell-free DNA reveals high concordance with metastatic tumors. Nat Commun 8, 1324 (2017).

Drews, R.M., Hernando, B., Tarabichi, M. et al. A pan-cancer compendium of chromosomal instability. Nature 606, 976-983 (2022).

Macintyre, G. et al. Copy number signatures and mutational processes in ovarian carcinoma. Nat. Genet. 50, 1262-1270 (2018).

Christopher Pockrandt and others, GenMap: ultra-fast computation of genome mappability, Bioinformatics, Volume 36, Issue 12, June 2020, Pages 3687-3692.

Mehran Karimzadeh and others, Umap and Bismap: quantifying genome and methylome mappability, Nucleic Acids Research, Volume 46, Issue 20, 16 November 2018, Page e120.

Li H, Durbin R. 2009. Fast and accurate short read alignment with Burrows-Wheeler transform. Bioinformatics 25: 1754-1760.

Li H. (2013) Aligning sequence reads, clone sequences and assembly contigs with BWA-MEM. arXiv:1303.3997v2

Seshan VE, Olshen A (2023). DNAcopy: DNA Copy Number Data Analysis. doi:10.18129/B9.bioc.DNAcopy, R package version 1.76.0, bioconductor.org/packages/DNAcopy.

Scheinin, I. et al. DNA copy number analysis of fresh and formalin-fixed specimens by shallow whole-genome sequencing with identification and exclusion of problematic regions in the genome assembly. Genome Res. 24, 2022-2032 (2014).

Madrid, L. et al. Predicting response to cytotoxic chemotherapy. bioRxiv 2023.01.28.525988 (2023) doi:10.1101/2023.01.28.525988.

Alexandrov, L. B. et al. The repertoire of mutational signatures in human cancer. Nature 578, 94-101 (2020).

Grün, B. & Leisch, F. FlexMix Version 2: Finite Mixtures with Concomitant Variables and Varying and Constant Parameters. J. Stat. Softw. 028, (2008).

Gerstung, M. et al. The evolutionary history of 2,658 cancers. Nature 578, 122-128 (2020).

Dentro, S. C. et al. Characterizing genetic intra-tumor heterogeneity across 2,658 human cancer genomes. Cell 184, 2239-2254.e39 (2021).

ICGC/TCGA Pan-Cancer Analysis of Whole Genomes Consortium. Pan-cancer analysis of whole genomes. Nature 578, 82-93 (2020).

Hübschmann, D. et al. Analysis of mutational signatures with yet another package for signature analysis. Genes Chromosomes Cancer 60, 314-331 (2021).

Li, H. et al. The Sequence Alignment/Map format and SAMtools. Bioinformatics 25, 2078-2079 (2009).

McGranahan, N., Burrell, R. A., Endesfelder, D., Novelli, M. R. & Swanton, C. Cancer chromosomal instability: therapeutic and diagnostic challenges. EMBO Rep. 13,528-538 (2012). Steele, C. D. et al. Signatures of copy number alterations in human cancer. Nature 606, 984-991 (2022).

All references cited herein are incorporated herein by reference in their entirety and for all purposes to the same extent as if each individual publication or patent or patent application was specifically and individually indicated to be incorporated by reference in its entirety.

The specific embodiments described herein are offered by way of example, not by way of limitation. Various modifications and variations of the described compositions, methods, and uses of the technology will be apparent to those skilled in the art without departing from the scope and spirit of the technology as described. Any sub-titles herein are included for convenience only and are not to be construed as limiting the disclosure in any way. Unless context dictates otherwise, the descriptions and definitions of the features set out above are not limited to any particular aspect or embodiment of the invention and apply equally to all aspects and embodiments which are described.

The methods of any embodiments described herein may be provided as computer programs or as computer program products or computer readable media carrying a computer program which is arranged, when run on a computer, to perform the method(s) described above.

Throughout the specification and claims, the following terms take the meanings explicitly associated herein, unless the context clearly dictates otherwise. The phrase "in one embodiment" as used herein does not necessarily refer to the same embodiment, though it may. Furthermore, the phrase "in another embodiment" as used herein does not necessarily refer to a different embodiment, although it may. Thus, as described below, various embodiments of the invention may be readily combined, without departing from the scope or spirit of the invention.

It must be noted that, as used In the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

Other aspects and embodiments of the invention provide the aspects and embodiments described above with the term "comprising" replaced by the term "consisting of" or "consisting essentially of", unless the context dictates otherwise. "and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

### Claims

1. A computer implemented method of characterising chromosomal instability in a DNA sample obtained from a tumour, the method comprising:

   obtaining a tumour copy number profile for the sample;
   quantifying, for each of one or more segments in the copy number profile, a set of copy number features, wherein the set of copy number features consists of copy number features that are associated with respective segments; and
   determining, for a segment of the one or more segments, a metric indicative of association between one or more signatures of chromosomal instability and the segment using the quantified features for the segment, wherein a signature of chromosomal instability comprises a set of weights for each of a plurality of components each associated with a copy number feature of the set of copy number features, wherein the weights are indicative of the probability that a mutational process associated with the signature generates a segment having characteristics defined by the component.

2. The method of claim 1, wherein the set of copy number features comprises or consists of: segment size, copy number change-point, and breakpoint count in one or both flanking regions of a predetermined length, and/or wherein determining a metric indicative of association between a signature of chromosomal instability and a segment comprises obtaining a value for each of the plurality of components for the segment, wherein each component defines characteristics of a segment as a predetermined distribution of values of a copy number feature.

3. The method of any preceding claim, wherein determining a metric indicative of association between a signature of chromosomal instability and a segment comprises determining the probability of the copy number features of the segment belonging to each of a set of predetermined distribution associated with respective components of the plurality of components.

4. The method of any preceding claim, wherein the set of copy number features for a segment comprises a summarised value of the breakpoint count in the upstream and downstream flanking regions of a predetermined length around the segment, optionally wherein the summarised value is the maximum and/or wherein the predetermined length is between 2 and 10 Mb, between 3 and 8 Mb, or about 5 Mb.

5. The method of any preceding claim, wherein each copy number feature is associated with a plurality of components, and each component defines a characteristic of copy number events using a respective predetermined distribution, wherein the predetermined distributions for a copy number feature that is quasi-continuous (e.g. segment size, copy number changepoint) is a set of Gaussian distributions, and/or wherein the predetermined distributions for any count feature (e.g. breakpoint count in one or both flanking regions of a predetermined length) is a set of Poisson distributions, optionally the predetermined distributions associated with the components are the distributions defined by the parameters in Table 1, or corresponding distributions obtained by fitting mixture models to values for the set of copy number features obtained from copy number profiles from a plurality of tumour samples, and/or wherein the predetermined distributions comprises between 15 and 25 Gaussian distributions for the segment size, between 5 and 15 Gaussian distributions for the copy number changepoint, and between 5 and 15 Poisson distributions for the breakpoint count in one or both flanking regions of a predetermined length.

6. The method of any preceding claim, wherein the set of copy number features comprises a copy number change-point, wherein the copy number changepoint of a current segment is determined by reference to the upstream segment of the current segment when the copy number changepoint relative to the upstream segment is at or above a predetermined threshold, by reference to the downstream segment of the current segment when the copy number changepoint relative to the upstream segment is below the predetermined threshold and the copy number change-point relative to the downstream segment is at or above the predetermined threshold, and by reference to a reference copy number value, optionally 2, when the copy number changepoints relative to the upstream and downstream segments are below the predetermined threshold, optionally wherein the predetermined threshold is -3.

7. The method of any preceding claim, wherein determining a metric indicative of association between a signature of chromosomal instability and a segment using the quantified features for the segment comprises multiplying probabilities associated with each of the plurality of components for the segment by the corresponding weight in the signature, and summing up the resulting values, optionally wherein the probability associated with a component for the segment is the probability of a segment with the quantified feature having been drawn from the distribution associated with the component.

8. The method of claim 10, wherein determining a metric indicative of association between a signature of chromosomal instability and a segment comprises for each of a plurality of signatures, multiplying probabilities associated with each of the plurality of components for the segment by the corresponding weight in the signature, and summing up the resulting values to obtain an exposure for the signature, and normalising the resulting exposures by dividing each exposure by the sum of exposures across the plurality of signatures.

9. The method of any of claims 1 to 8, wherein determining a metric indicative of association between a signature of chromosomal instability and a segment using the quantified features for the segment comprises obtaining probabilities associated with each of the plurality of components for the segment and determining a distance between said probabilities and the weights of the signature, optionally wherein the distance is a cosine distance or a Euclidian distance.

10. The method of any preceding claim, further comprising assigning a signature of chromosomal instability to a segment of the one or more segments as the signature of the one or more signatures of chromosomal instability with the highest value of the metric for the segment, and/or wherein the metric for a signature is a probability that the segment is associated with the signature.

11. The method of any preceding claim, wherein the one or more signatures of chromosomal instability are selected from those defined in Table 3 or corresponding signatures obtained by quantifying the set of copy number features in a plurality of tumour samples, and identifying one or more signatures likely to result in the copy number profiles of the plurality of tumour samples by nonnegative matrix factorisation, and/or wherein the one or more signatures comprise one or more signatures selected from: one or more signatures associated with chromosome missegregation, optionally chromosome missegregation via defective mitosis and/or telomere dysfunction, one or more signatures associated with impaired homologous recombination, one or more signatures associated with tolerance of whole genome duplication, one or more signatures associated with impaired non-homologous end joining, one or more signatures associated with replication stress, and/or one or more signature associated with impaired DNA damage sensing.

12. The method of any preceding claim, further comprising determining that the copy number profile comprises a number of non-diploid segments above a predetermined threshold, and/or

wherein the copy number profile comprises at least 15 non-diploid segments, and/or
wherein obtaining the tumour copy number profile comprises determining the tumour copy number profile from sequence data, optionally wherein the sequence data is next-generation sequencing data and/or wherein determining the tumour copy number profile comprises determining a copy number for each of a plurality of genomic bins of a predetermined size, and/or
wherein the method comprises obtaining a tumour copy number profile from DNA sequence data comprising a plurality of sequence read, wherein the tumour copy number profile comprises a copy number estimate for each of a plurality of genomic bins comprising a number of mapped reads above a predetermined threshold.

13. A method of predicting whether a subject with cancer is likely to respond to a therapy, the method comprising: characterising a DNA sample obtained from a tumour of the subject, using the method of any of claims 1 to 12, as having at least one segment with a metric associated with one or more predetermined signatures of chromosomal instability satisfying a predetermined criterion and/or as having a sample exposure to said signatures satisfying a predetermined criterion, wherein: (a) the predetermined signatures comprise a signature associated with response to inhibition of a particular gene, the therapy is a therapy that targets the gene, and when the sample is characterised as having at least one segment with the metric associated with the signature satisfying a predetermined criterion and/or as having a sample exposure to said signature satisfying a predetermined criterion, the subject is likely to respond to the therapy; or (b) the therapy is a therapy for which resistance to the therapy is associated with amplification of a gene, optionally amplification of an oncogene, when the sample is characterised as having at least one segment with the metric associated with the one or more signatures satisfying a predetermined criterion and/or as having a sample exposure to said signatures satisfying a predetermined criterion, the subject is likely to develop an amplification of the gene and is unlikely to respond to the therapy.

14. A method of determining whether a subject with cancer is likely to develop a gene amplification, the method comprising:
characterising a DNA sample obtained from a tumour of the subject, using the method of any of claims 1 to 12, as having at least one segment with a metric associated with one or more predetermined signatures of chromosomal instability satisfying a predetermined criterion and/or as having a sample exposure to said signatures satisfying a predetermined criterion;

wherein the presence of at least one segment with a metric associated with the one or more predetermined signatures of chromosomal instability satisfying a predetermined criterion and/or as having a sample exposure to said signatures satisfying a predetermined criterion indicates that the subject is likely to develop an amplification of a gene;
optionally wherein the gene is an oncogene.

15. A method of determining whether a particular process causing chromosomal instability is currently active in a subject's cancer, the method comprising:

characterising a sample obtained from said subject using the method of any of claims 1 to 12, wherein the tumour copy number profiles are obtained from single cell sequencing data and the characterising is performed individually using each of a plurality of single cell tumour copy number profiles;
identifying one or more shared copy number events as events that are present in a proportion of single cell tumour copy number profiles above a predetermined threshold and/or one or more unique copy number events that are present in a proportion of single cell tumour copy number profiles at or below the predetermined threshold;
wherein a process causing chromosomal instability associated with a signature that has been determined to be associated with a shared copy number event is likely to have been active in the tumour prior to at least one clonal expansion in the tumour, and/or a process causing chromosomal instability associated with a signature that has been determined to be associated with a unique copy number event is likely to be currently active in the tumour.

```
┌ ─ ─ ─ ─ ─ ─ ─ ─ ─ ┐
:  Obtain sample  :── 10
└ ─ ─ ─ ─ ─ ─ ─ ─ ─ ┘
          │
          ▼
┌ ─ ─ ─ ─ ─ ─ ─ ─ ─ ─ ─ ─ ┐
:  Obtain sequence data  :── 12
└ ─ ─ ─ ─ ─ ─ ─ ─ ─ ─ ─ ─ ┘
          │
          ▼
┌─────────────────────────────────────────────┐
│ Obtain tumour copy number profile for the sample │── 14
└─────────────────────────────────────────────┘
          │
          ▼                                      ╱ 16
┌─────────────────────────────────────────────┐
│ Quantify set of copy number features of non-diploid │
│    segments (events)  in copy number profile    │
└─────────────────────────────────────────────┘
          │
          ▼                                      ╱ 18
┌─────────────────────────────────────────────┐
│      Determine event-specific association with      │
│       signature(s) of chromosomal instability       │
└─────────────────────────────────────────────┘
          │
          ▼                                      ╱ 20
┌ ─ ─ ─ ─ ─ ─ ─ ─ ─ ─ ─ ─ ─ ─ ─ ─ ─ ─ ─ ─ ─ ┐
: Determine sample-level exposure to signature(s) of :
:             chromosomal instability             :
└ ─ ─ ─ ─ ─ ─ ─ ─ ─ ─ ─ ─ ─ ─ ─ ─ ─ ─ ─ ─ ─ ┘
          │
          ▼                                      ╱ 22
┌ ─ ─ ─ ─ ─ ─ ─ ─ ─ ─ ─ ─ ─ ─ ─ ─ ─ ─ ─ ─ ─ ┐
: Determine presence / absence of signature(s) and/or :
:              processes causing CIN              :
└ ─ ─ ─ ─ ─ ─ ─ ─ ─ ─ ─ ─ ─ ─ ─ ─ ─ ─ ─ ─ ─ ┘
          │
          ▼
┌ ─ ─ ─ ─ ─ ─ ─ ─ ─ ─ ─ ─ ─ ┐
:  Provide results to user  :── 24
└ ─ ─ ─ ─ ─ ─ ─ ─ ─ ─ ─ ─ ─ ┘
```

Fig. 1

Fig. 3

Fig. 3

Figure 4

Figure 5

Figure 6

**Activities of novel CIN signatures**

| | CX1 | CX2 | CX3 | CX4 | CX5 | CX7 | CX8 | CX9 | CX10 | CX11 | CX12 | CX13 | CX14 | CX15 | CX16 | CX17 | |
|---|---|---|---|---|---|---|---|---|---|---|---|---|---|---|---|---|---|
| | 0.745 | -0.377 | -0.587 | -0.182 | -0.475 | -0.244 | -0.130 | -0.225 | -0.268 | -0.176 | -0.128 | -0.123 | 0.559 | -0.146 | -0.109 | -0.115 | CX1 |
| | 0.085 | 0.640 | -0.042 | -0.510 | -0.196 | -0.252 | -0.276 | -0.131 | -0.058 | 0.093 | 0.055 | -0.124 | -0.080 | -0.114 | -0.095 | -0.074 | CX2 |
| | 0.050 | -0.212 | 0.439 | -0.330 | -0.447 | -0.013 | -0.145 | -0.170 | -0.165 | -0.183 | -0.123 | -0.227 | 0.089 | 0.023 | -0.076 | -0.154 | CX3 |
| | 0.079 | -0.382 | -0.081 | 0.748 | -0.086 | -0.223 | 0.069 | -0.061 | -0.002 | -0.116 | -0.005 | 0.015 | -0.086 | -0.155 | 0.005 | 0.139 | CX4 |
| | -0.602 | 0.395 | 0.117 | 0.355 | 0.861 | 0.332 | 0.329 | 0.350 | 0.158 | 0.031 | 0.078 | 0.266 | -0.502 | 0.098 | 0.072 | 0.144 | CX5 |
| | 0.022 | -0.244 | 0.071 | -0.294 | 0.044 | 0.623 | -0.134 | -0.026 | -0.106 | 0.038 | -0.013 | -0.080 | 0.211 | 0.249 | -0.039 | -0.223 | CX7 |
| | -0.014 | -0.190 | -0.026 | 0.111 | 0.047 | -0.090 | 0.753 | 0.091 | 0.067 | -0.045 | -0.073 | 0.042 | -0.080 | -0.067 | 0.141 | 0.128 | CX8 |
| | -0.105 | 0.115 | -0.150 | 0.130 | 0.262 | -0.043 | 0.114 | 0.628 | 0.145 | 0.222 | 0.082 | 0.239 | -0.138 | -0.087 | -0.018 | -0.033 | CX9 |
| | -0.136 | 0.118 | -0.007 | 0.044 | 0.078 | -0.053 | 0.031 | 0.210 | 0.704 | 0.226 | -0.135 | 0.064 | -0.088 | -0.127 | -0.031 | -0.063 | CX10 |
| | 0.039 | 0.128 | -0.174 | -0.070 | -0.030 | -0.010 | -0.024 | 0.188 | 0.105 | 0.714 | 0.100 | 0.254 | 0.017 | -0.098 | -0.054 | -0.084 | CX11 |
| | 0.093 | -0.007 | -0.097 | -0.090 | -0.089 | -0.044 | -0.058 | -0.033 | -0.072 | 0.094 | 0.727 | 0.010 | 0.066 | -0.072 | 0.239 | -0.027 | CX12 |
| | -0.038 | -0.006 | -0.138 | 0.075 | 0.164 | -0.031 | 0.018 | 0.358 | 0.042 | 0.348 | 0.052 | 0.882 | -0.059 | -0.084 | -0.018 | 0.018 | CX13 |
| | 0.637 | -0.425 | -0.471 | -0.258 | -0.432 | -0.156 | -0.173 | -0.207 | -0.201 | -0.166 | -0.128 | -0.143 | 0.810 | -0.026 | -0.104 | -0.144 | CX14 |
| | -0.035 | -0.106 | 0.124 | -0.225 | 0.045 | 0.250 | -0.059 | -0.198 | -0.154 | -0.111 | -0.068 | -0.086 | 0.074 | 0.513 | -0.029 | -0.174 | CX15 |
| | -0.265 | 0.006 | 0.237 | 0.060 | 0.097 | 0.056 | 0.101 | 0.029 | 0.006 | 0.109 | 0.344 | -0.010 | -0.190 | -0.011 | 0.758 | 0.075 | CX16 |
| | -0.035 | -0.185 | 0.109 | 0.183 | -0.093 | -0.066 | 0.026 | -0.096 | -0.051 | -0.106 | -0.015 | -0.067 | -0.080 | -0.118 | -0.011 | 0.403 | CX17 |

**Activities of original CIN signatures**

Pearson's r
1
0.5
0
-0.5
-1

Figure 7

Figure 8

Figure 9

Figure 10

Fig. 11

Fig. 12

3 dominant sigs - 1000 samples & 1000 runs
Minimum number of events for having >90% cases correctly detected: 15

Fig. 13

Fig. 14

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/057392 A1 (CAMBRIDGE ENTPR LTD [GB] ET AL.) 13 April 2023 (2023-04-13) | 1 | INV.<br>G16B20/10<br>C12Q1/6886<br>G16B40/00 |
| A | * claim 1 to 8 * | 2-15 | |

-----

**TECHNICAL FIELDS SEARCHED (IPC)**

G16B
C12Q

The present search report has been drawn up for all claims

| Place of search | Date of completion of the search | Examiner |
|---|---|---|
| Munich | 21 June 2024 | Heidrich, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

# EP 4 557 298 A1

## ANNEX TO THE EUROPEAN SEARCH REPORT
## ON EUROPEAN PATENT APPLICATION NO.

EP 23 38 3179

This annex lists the patent family members relating to the patent documents cited in the above-mentioned European search report.
The members are as contained in the European Patent Office EDP file on
The European Patent Office is in no way liable for these particulars which are merely given for the purpose of information.

21-06-2024

| Patent document cited in search report | Publication date | Patent family member(s) | | Publication date |
|---|---|---|---|---|
| WO 2023057392 A1 | 13-04-2023 | AU | 2022358908 A1 | 11-04-2024 |
| | | CA | 3233198 A1 | 13-04-2023 |
| | | WO | 2023057392 A1 | 13-04-2023 |

EPO FORM P0459

For more details about this annex : see Official Journal of the European Patent Office, No. 12/82

49

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2023057392 A

**Non-patent literature cited in the description**

- **RAINE KM et al.** Identifying Somatically Acquired Copy-Number Alterations from Whole-Genome Sequencing Data. *Curr Protoc Bioinformatics*, 08 December 2016, vol. 56, 15.9.1-15.9.17
- **VAN LOO, P. et al.** Allele-specific copy number analysis of tumors. *Proc. Natl. Acad. Sci. U. S. A.*, 2010, vol. 107, 16910-16915
- **LANGMEAD, B** ; **TRAPNELL, C.** ; **POP, M. et al.** Ultrafast and memory-efficient alignment of short DNA sequences to the human genome. *Genome Biol*, 2009, vol. 10, R25
- **CARTER, S.** ; **CIBULSKIS, K.** ; **HELMAN, E et al.** Absolute quantification of somatic DNA alterations in human cancer. *Nat Biotechnol*, 2012, vol. 30, 413-421
- **FAVERO F** ; **JOSHI T** ; **MARQUARD AM** ; **BIRKBAK NJ** ; **KRZYSTANEK M** ; **LI Q** ; **SZALLASI Z** ; **EKLUND AC**. Sequenza: allele-specific copy number and mutation profiles from tumor sequencing data. *Ann Oncol.*, January 2015, vol. 26 (1), 64-70
- **ADALSTEINSSON, V.A** ; **HA, G.** ; **FREEMAN, S.S et al.** Scalable whole-exome sequencing of cell-free DNA reveals high concordance with metastatic tumors. *Nat Commun*, 2017, vol. 8, 1324
- **DREWS, R.M** ; **HERNANDO, B.** ; **TARABICHI, M et al.** A pan-cancer compendium of chromosomal instability. *Nature*, 2022, vol. 606, 976-983
- **MACINTYRE, G et al.** Copy number signatures and mutational processes in ovarian carcinoma. *Nat. Genet*, 2018, vol. 50, 1262-1270
- **CHRISTOPHER POCKRANDT**. GenMap: ultra-fast computation of genome mappability. *Bioinformatics*, June 2020, vol. 36 (12), 3687-3692
- **MEHRAN KARIMZADEH**. Umap and Bismap: quantifying genome and methylome mappability. *Nucleic Acids Research*, 16 November 2018, vol. 46 (20), e120
- **LI H** ; **DURBIN R**. Fast and accurate short read alignment with Burrows-Wheeler transform. *Bioinformatics*, 2009, vol. 25, 1754-1760
- **LI H.** Aligning sequence reads, clone sequences and assembly contigs with BWA-MEM. *arXiv:1303.3997v2*, 2013

- **SESHAN VE** ; **OLSHEN A**. DNAcopy: DNA Copy Number Data Analysis. doi:10.18129/B9.bioc.DNAcopy. *R package version 1.76.0, bioconductor.org/packages/DNAcopy.*, 2023
- **SCHEININ, I et al.** DNA copy number analysis of fresh and formalin-fixed specimens by shallow whole-genome sequencing with identification and exclusion of problematic regions in the genome assembly. *Genome Res*, 2014, vol. 24, 2022-2032
- **MADRID, L. et al.** Predicting response to cytotoxic chemotherapy. *bioRxiv 2023.01.28.525988*, 2023
- **ALEXANDROV, L. B. et al.** The repertoire of mutational signatures in human cancer. *Nature*, 2020, vol. 578, 94-101
- **GRÜN, B** ; **LEISCH, F**. FlexMix Version 2: Finite Mixtures with Concomitant Variables and Varying and Constant Parameters. *J. Stat. Softw. 028*, 2008
- **GERSTUNG, M. et al.** The evolutionary history of 2,658 cancers.. *Nature*, 2020, vol. 578, 122-128
- **DENTRO, S. C. et al.** Characterizing genetic intra-tumor heterogeneity across 2,658 human cancer genomes. *Cell*, 2021, vol. 184, 2239-2254, e39
- ICGC/TCGA Pan-Cancer Analysis of Whole Genomes Consortium. Pan-cancer analysis of whole genomes. *Nature*, 2020, vol. 578, 82-93
- **HÜBSCHMANN, D. et al.** Analysis of mutational signatures with yet another package for signature analysis.. *Genes Chromosomes Cancer*, 2021, vol. 60, 314-331
- **LI, H. et al.** The Sequence Alignment/Map format and SAMtools. *Bioinformatics*, 2009, vol. 25, 2078-2079
- **MCGRANAHAN, N.** ; **BURRELL, R. A.** ; **ENDES-FELDER, D.** ; **NOVELLI, M. R** ; **SWANTON, C**. Cancer chromosomal instability: therapeutic and diagnostic challenges. *EMBO Rep*, 2012, vol. 13, 528-538

- **STEELE, C. D. et al.** Signatures of copy number alterations in human cancer. *Nature*, 2022, vol. 606, 984-991